# EUROPÄISCHE PATENTSCHRIFT

(11) **EP 2 857 053 B1**
(45) Veröffentlichungstag und Bekanntmachung des Hinweises auf die Patenterteilung: **06.12.2017**
(21) Anmeldenummer: 14187780.3
(22) Anmeldetag: 06.10.2014
(51) Int. Cl.: A61M 1/14, A61M 1/34, A61B 5/11, G06F 3/01, G06F 19/00

(54) **Dialysevorrichtung mit Bewegungserfassungseinrichtung und Gestensteuerung**
Dialysis device with movement detection device and gesture control
Dispositif de dialyse doté d'un dispositif de détection de mouvement et de commande de geste

(30) Priorität: 07.10.2013 DE 102013111084
(43) Veröffentlichungstag der Anmeldung: 08.04.2015
(73) Patentinhaber: B. Braun Avitum AG, 34212 Melsungen (DE)
(72) Erfinder: Ritter, Kai-Uwe, 91126 Rednitzhembach (DE); Bröker, Björn, 34355 Staufenberg (DE)
(74) Vertreter: Winter, Brandl, Fürniss, Hübner, Röss, Kaiser, Polte - Partnerschaft mbB

(56) Entgegenhaltungen:
- EP-A2- 1 574 178
- WO-A1-2009/094183
- WO-A1-2014/063798
- WO-A1-2014/159022
- DE-A1-102011 053 935
- US-A1- 2003 128 125
- US-A1- 2010 137 693
- US-A1- 2011 157 480

## Beschreibung

Die Erfindung betrifft eine Dialysevorrichtung mit einer Bewegungserfassungseinrichtung und Gestensteuerung, und bezieht sich insbesondere auf eine Dialysevorrichtung, ein Dialysegerät, eine Dialysemaschine und dergleichen mit zumindest einer Bewegungserfassungseinrichtung in Form beispielsweise einer Kamera oder einer Sensoranordnung, die dazu ausgelegt ist, Bewegungen, Gesten und/oder Handlungen einer Person in einem Sichtbereich der Bewegungserfassungseinrichtung zu überwachen, aufzuzeichnen und/oder zu übertragen.

Derzeit verfügbare Dialysegeräte werden ausschließlich durch Tasten und/oder Eingaben an einem berührungsempfindlichen Bildschirm (beispielsweise einem Touchscreen) bedient. Ein Anwender muss somit das Gerät unter Umständen mit nassen oder kontaminierten Fingern bedienen, so dass regelmäßige Oberflächendesinfektion zur Vermeidung von Kreuzkontamination erforderlich ist. Außerdem unterstützen solche Geräte Bedienabläufe an einem Patienten nur bedingt, weil die Aufmerksamkeit eines Anwenders regelmäßig von dem Patienten zu einer Benutzerschnittstelle des Gerätes wechselt, um bestimmte Sequenzen des Anlegens und Ablegens des Patienten auszuführen. Beispielsweise müssen etwa von dem Gerät ausgegebene Alarme und dergleichen zunächst durch Tastendruck stillgeschaltet und nach Behebung der Alarm auslösenden Ursache (meist am Patienten und/oder einem Schlauchsystem des Geräts) am Bildschirm quittiert werden.

Weiter kann vorgesehen sein, dass ein Patient über eine Rufeinrichtung zum Herbeirufen von Personal verfügt. Eine solche Rufeinrichtung wird jedoch bislang als separat an das Gerät angebundene Vorrichtung dargestellt, denn naturgemäß werden einem Patienten Bedienvorgänge direkt am Gerät selbst entweder gänzlich untersagt, oder sind solche Bedienvorgänge einem Patienten, etwa im Rahmen einer Limited-Care-Dialyse, aufgrund der Sitz- und/oder Liegeposition während der Behandlung ohnehin kaum möglich.

Beispielsweise sind für Dialysetherapien oder Dialysebehandlungen häufige Wiegevorgänge vor, während und nach der eigentlichen Dialyse durchzuführen. Bei Dialysepatienten liegt eine Unterfunktion der natürlichen Niere vor. Deren Aufgaben, etwa die Entfernung urämischer Toxine und die Ausscheidung von überschüssigem Körperwasser, muss sodann eine künstliche Niere (ein Dialysator) übernehmen. In einer Ausführungsform der künstlichen Niere presst diese während ihres Blutreinigungsvorgangs Plasmawasser des Patienten über eine Dialysatormembran ab. Dieses Abpressen von Plasmawasser, oder diese Wasserentfernung durch Filtration des Bluts über eine Siebmembran mit einem Druck von 0 bis 1 bar und mit dabei auftretenden Mitnahmeeffekten (Konvektion) zur selektiven Entgiftung, wird als Ultrafiltration bezeichnet. Mittels der Ultrafiltration ist es möglich, Teilchen im Bereich der Größe mittelmolekularer Substanzen (etwa 0,1 bis 0,05 µm), wie beispielsweise Endotoxine, Viren, Kolloide, Proteine und Enzyme aus dem Blut des Patienten zu entfernen bzw. zu waschen und es dadurch zu reinigen. Da diese Reinigung außerhalb des Körpers des Patienten erfolgt, verliert der Körper dabei zugleich Wasser und beispielsweise Elektrolyte, die ersetzt werden müssen. Ferner können je nach Durchlässigkeit der Filter große Filtratmengen in kurzer Zeit entstehen, die den notwendigen Volumenentzug des Patienten übersteigen. Eine der Filtratmenge entsprechende Substitution von Flüssigkeit physiologischer Zusammensetzung ist daher erforderlich, um den Flüssigkeitshaushalt des Patienten zu stabilisieren.

Im Gegensatz zu Menschen mit gesunden Nieren ist bei Nierenkranken die Fähigkeit zur Regulierung des Wasserhaushalts im Körper mehr oder weniger stark gestört, so dass überschüssiges Wasser im Körper zurückgehalten wird. Dadurch kommt es zu einer Mehrbelastung des Organismus als einer der Hauptfaktoren, die für Dialysepatienten eine Zunahme an Komplikationen und eine Abnahme an Lebenserwartung bedeuten.

Eine der wichtigsten Aufgaben der Dialysebehandlung besteht daher darin, die fehlende Regulierung des Wasserhaushalts so gut wie möglich zu ersetzen. Üblicherweise wird dazu bei Dialysebehandlungen ein sogenanntes Trockengewicht als Behandlungsziel festgelegt. Ein Definitionsvorschlag für das Trockengewicht zieht einen Schätzwert für das Gewicht heran, das ein Dialysepatient hätte, wenn er/sie keine Nierenerkrankung hätte. Medizinisch gesehen wird als Trockengewicht das Gewicht zum Ende der Dialyse bezeichnet, bei dem der Patient keine Überwässerungsanzeichen aufweist, frei von Symptomen ist, sich wohlfühlt und bei dem bis zur nächsten Dialyse ein normaler Blutdruck erhalten bleibt.

Zur Bestimmung des Trockengewichts, das als solches nicht durch das Ergebnis eines objektiven Vorgangs einfach festgelegt werden kann, sind möglichst exakte Wiegevorgänge des Patienten vor und nach der Dialysebehandlung unerlässlich, um die Ultrafiltrationsmenge so bemessen zu können, dass als Sollgewicht das Trockengewicht möglichst genau erreicht wird. Aus Beobachtungen ist aber bekannt, dass immer wieder unerklärliche und unerwünschte Abweichungen der Ultrafiltrationsmenge beim Wiegen oder zwischen einzelnen Wiegevorgängen festgestellt werden. Um in solchen Fällen ausschließen zu können, dass die Dialysemaschinen die Ursache solcher Abweichungen sind, müssen diese zeitaufwändig und kostenträchtig außer Betrieb genommen und in einer simulierten Behandlung auf ihre Genauigkeit geprüft werden. Häufig stellt sich dann heraus, dass die Funktion der Maschine nicht zu beanstanden ist.

Die Ursache für eine Abweichung kann dann beispielsweise auf derzeit noch nicht nachvollziehbare Ursachen zurückzuführen sein. Beispiele für eine Vielzahl derartiger Ursachen sind, dass sich der Patient zwischen Wiegevorgängen erleichtert hat, dies aber nicht ordnungsgemäß vermerkt wurde; der Patient absichtlich ein falsches Gewicht meldet, mit dem Ziel, sich mehr Flüssigkeit entziehen zu lassen, um danach mehr als zugestanden trinken zu können; eine Nahrungs- und/oder Getränkeaufnahme nicht protokolliert wurde; eine während der Behandlung verabreichte Infusion nicht genau gemessen und/oder nicht protokolliert wurde; der Patient bei zwei einander zuzuordnenden Wiegevorgängen nicht dieselbe Kleidung trägt; der Patient bei zwei einander zuzuordnenden Wiegevorgängen unterschiedliche Rollstühle mit jeweils anderem Gewicht benutzt; beim Wiegen kurzzeitig Gegenstände (eine Tasche, ein Mobiltelefon oder dergleichen) abgelegt oder nicht abgelegt wurden; ein benutzter Rollstuhl einen nicht optimalen Stand hatte; oder sich der Patient während des Wiegevorgangs abstützt.

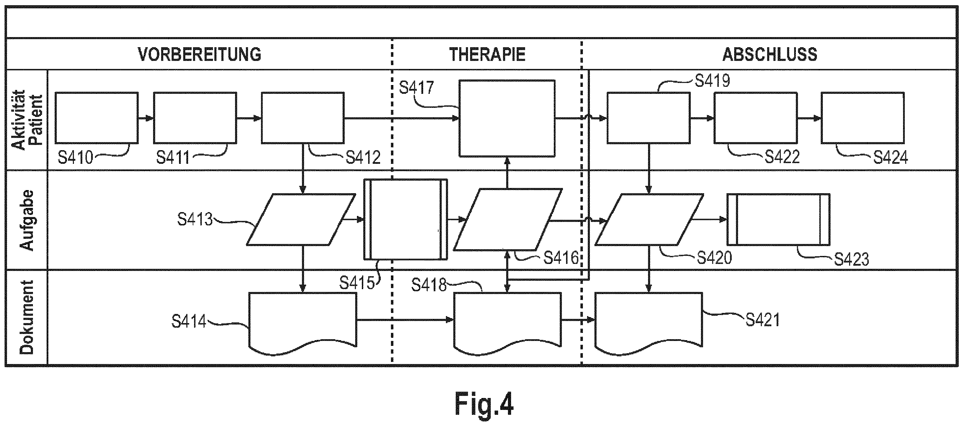

Fig. 4 zeigt beispielsweise eine vereinfachte Darstellung eines bekannten Ablaufs eines Wiegevorgangs unter Verwendung einer Protokollierung erfasster Daten auf Papier. Im Einzelnen sind in dem gezeigten zeitlichen Ablauf in chronologischer Abfolge Schritte oder Aktivitäten angegeben, die während des Wiegevorgangs eines Dialysepatienten im Rahmen eines Therapieablaufs mit vorbereitendem Wiegen vor der Therapie, der Therapie basierend auf den während des Wiegens ermittelten Daten, und dem Abschluss der Therapie mit erneutem kontrollierendem Wiegen von dem Patienten und Personal einer Dialysestation durchzuführen sind. Insoweit Aktivitäten und/oder Schritte parallel und/oder gleichzeitig durchzuführen sind, ist die Nummerierung der einzelnen Schritte nicht zwingend als Abfolge oder Aufeinanderfolge zu verstehen, sondern kann aus Zweckmäßigkeitsgründen auch nur eine Angabe von Bezugszeichen zur eindeutigen Zuordnung darstellen.

Die in Fig. 4 während des zeitlichen Ablaufs des Wiegevorgangs von links nach rechts untereinander in den einzelnen Zeilen angegebenen Bezeichnungen, d. h. "Aktivität Patient", "Aufgabe" und "Dokument", können eine variable Zuordnung von Aktivitäten zu Patient und/oder Personal beinhalten. Beispielsweise kann ein Patient eine Protokollierungsaktivität durchführen, oder kann Personal diese Protokollierungsaktivität durchführen.

Gemäß Fig. 4 beginnt ein Dialysetherapieablauf damit, dass ein Patient in einem Schritt S410 im Dialysezentrum eintrifft. In einem Schritt S411 legt er zumindest teilweise seine Kleidung, beispielsweise eine Jacke, und gegebenenfalls mitgeführtes Gepäck in einer zum Erhalt einer definierten Ausgangssituation üblicherweise vorbestimmten Weise ab. In einem Schritt S412 erfolgt der Wiegevorgang, während dem in einem Schritt S413 ein derzeitiges Ist-Gewicht des Patienten ermittelt wird. Hierbei kann sich der Patient selbst wiegen oder sich von Personal wiegen lassen. In einem Schritt S414 wird dieses Ist-Gewicht durch beispielsweise Personal der Dialysestation in ein Wiegeprotokoll eingetragen. Das Wiegeprotokoll wird während einer Dialysetherapie fortgeführt und kann in deren Rahmen durchgeführte mehrere Wiegevorgänge beinhalten. Darüber hinaus erfolgt die Protokollierung von Therapiedaten und somit auch Wiegevorgängen über mehrere zeitlich getrennte Dialysebehandlungen hinweg. Nach der Ermittlung des Ist-Gewichts des Patienten in Schritt S413 wird dieses die Dialysebehandlung vorbereitend dazu herangezogen, in einem Schritt S415 eine Soll-Ultrafiltrationsmenge bzw. ein aktuelles Ist-Trockengewicht zu bestimmen. Darauf basierende Therapiedaten bzw. Behandlungsparameter werden sodann in einem Schritt S416 von Personal in eine Dialysemaschine eingegeben bzw. in diese übertragen, und in einem Schritt S417 wird mit diesen Therapiedaten nach dem Anschluss des Patienten an die Dialysemaschine die Dialysebehandlung mit den festgelegten bzw. verschriebenen Therapiedaten durchgeführt. Möglichst gleichzeitig bzw. parallel dazu wird der Therapieablauf oder Therapieverlauf von Personal in einem Schritt S418 mitprotokolliert. Nach dem Ende der Dialysetherapie wird der Patient von der Dialysemaschine abgeschlossen und sodann in einem Schritt S419 erneut gewogen. Erneut werden während des Wiegevorgangs in einem Schritt S420 möglichst zeitgleich oder parallel dazu das Ist-Gewicht bzw. das Ist-Trockengewicht des Patienten ermittelt und in einem Schritt S421, im Rahmen einer Dokumentation der Behandlung, wieder in das mitgeführte Protokoll eingetragen und/oder in einem Schritt S423 in eine Datenbank, beispielsweise eine elektronische Patientendatenbank oder Patientenakte, eingetragen. Nach dem Wiegevorgang kann sich der Patient in einem Schritt S422 wieder ankleiden und sodann in einem Schritt S424 das Dialysezentrum verlassen.

Da bei der während des Therapieablaufs gemäß Fig. 4 durchgeführten rein manuellen Dokumentation das Personal neben der Betreuung der Patienten noch die Aufgabe hat, die Dokumentation durchzuführen, d. h. auf Papier auszufüllen und/oder in andere elektronische Geräte zu übertragen, ist es aufgrund der vielen zeitlich konzentrierten Abläufe für das Personal schwierig, sich an Einzelheiten zu erinnern. Die manuelle Dokumentation der Therapie durch Personal und/oder den Patienten selbst unterliegt daher unter anderem den vorstehend beispielhaft genannten Fehlermöglichkeiten und dadurch bedingten Abweichungen.

Von der Anmelderin selbst existiert ein aus dem Stand der Technik bekanntes System zur Überwachung und mit einer Datenbank. Dieses Überwachungssystem ist eine Software zur transparenten Abbildung und Steuerung zahlreicher Vorgänge einer Dialysebehandlung. Die generierten Daten angeschlossener Dialysegeräte, Analysegeräte (z. B. Blutgasanalyse) und Patientenwaagen werden automatisch in das Überwachungssystem übertragen und gespeichert. Dort werden sie visualisiert und stehen zur Bearbeitung zur Verfügung. Durch den bidirektionalen Datentransfer zwischen dem bekannten Überwachungssystem und den angeschlossenen Dialysegeräten sind die Datensätze auch während der Behandlung konsistent und abrufbar.

Bekannte Anordnungen auf diesem Gebiet können durch eine Vernetzung von Einzelsystemen wie beispielsweise Maschinen, Waagen und Medikation zwar eine gewisse Unterstützung des Ablaufs auf einer Dialysestation bereitstellen. Sie sind nachteilig jedoch nicht in der Lage, nachvollziehbaren Aufschluss über während des Wiegens von Patienten auftretende Abweichungen ermittelter Patientengewichte und/oder Ultrafiltrationsmengen zu geben. Sie sind ebenfalls nachteilig weiter nicht in der Lage, die Bedienung des Geräts durch einen Anwender (d. h. einen Patienten und/oder Personal der behandelnden Einrichtung) in der Umgebung des Geräts transparenter zu gestalten.

Aus der US 2003/0128125 A1 sind auf dem Gebiet der extrakorporalen Blutbehandlung ein Verfahren und eine Vorrichtung zur Maschinenfehlererfassung mit geringerer Anzahl von Falschpositiven durch Kombinieren mehrerer Sensoreingaben bekannt. Die Vorrichtung weist zumindest eine Bewegungserfassungseinrichtung, eine Steuereinheit zur Steuerung einer Dialysemaschine, und eine Schnittstelleneinheit zur Anzeige von Betriebsinformation, zur Bedienung und/oder zur Netzwerkkommunikation der Dialysemaschine auf. Die Steuereinheit ist dazu angeordnet, auf der Grundlage von Information über eine erfasste Bewegung Anweisungen zur Steuerung der Dialysemaschine zu erzeugen und an steuerbare Komponenten der Dialysemaschine auszugeben und Daten betreffend eine Anzeige von Betriebsinformation, eine Bedienung und/oder eine Netzwerkkommunikation mit der Schnittstelleneinheit auszutauschen. Die Schnittstelleneinheit ist dazu angeordnet, auf der Grundlage der ausgetauschten Daten und in Übereinstimmung mit der Information über die erfasste Bewegung die Anweisungen zur Steuerung der Dialysemaschine visuell nachzuführen und/oder eine Netzwerkkommunikation durchzuführen. Bei einer im Zusammenhang erfolgenden Bild- und Videoverarbeitung von Aufnahmen, die mittels einer sich außerhalb der Dialysemaschine bzw. deren Gehäuse befindenden Kamera erhalten wurden, sind das Gesicht in einem Sichtbereich der Kamera oder Änderungen im Gesichtsausdruck oder der Körperposition eines Patienten erkennbar und klassifizierbar.

Ferner bezieht sich die US 2011/0157480 A1 auf ein Integrationssystem für medizinische Instrumente mit Fernbedienbarkeit und offenbart eine alternativ gestenbasierte Steuerung durch einen Arzt oder eine Bedienperson in einem sterilen Umfeld. Dazu können Handbewegungen, Fingerzeige, Gestik und dergleichen erfasst, gelesen oder erkannt werden, um eine Anzeige von Bildern auf einer Anzeigeeinrichtung zu steuern, und können Gestik repräsentierende Daten erzeugt und zur Steuerung des Systems verarbeitet bzw. ausführbar herangezogen werden. Ferner kann durch die gestenbasierte Steuervorrichtung ein Betrieb einer graphischen Benutzerschnittstelle erfolgen, und etwa ein Cursor oder Zeiger in grundlegender Synchronisation dazu bewegt oder eine dargestellte Taste gedrückt werden.

Der Erfindung liegt daher als eine Aufgabe zugrunde, eine Dialysevorrichtung mit einer Bewegungserfassungseinrichtung zu schaffen, die in der Lage ist, Bewegungen in einem Sichtbereich der Bewegungserfassungseinrichtung derart zu erfassen, dass eine Umsetzung der Bewegungen in ausführbare Aktionen zur Steuerung der Dialysevorrichtung oder Komponenten derselben erfolgen kann.

Diese Aufgabe wird erfindungsgemäß durch die Merkmalskombination des Anspruchs 1 gelöst. Vorteilhafte Weiterbildungen der Erfindung sind Gegenstand der beigefügten Unteransprüche.

Ein Grundgedanke der Erfindung besteht somit darin, einerseits einen Patienten während seiner Dialysebehandlung mittels zumindest einer Kamera, einem Sensor oder dergleichen beobachten zu können. Die Kamera ist über eine Netzwerkverbindung mit einem Überwachungssystem verbunden, in welchem die gesamten erfassten Vorgänge gespeichert werden. Die von der Kamera erfassten und gelieferten Informationen und Daten werden mittels Steuerungs- und/oder Verarbeitungsroutinen, beispielsweise in Form von Software, analysiert, um zumindest Bewegungen des Patienten, eine Nahrungsaufnahme desselben und dergleichen zu erkennen, und/oder bilden eine Grundlage für eine Qualitätssicherung von Maßnahmen während der Dialyse (beispielsweise ob Medikamente verabreicht und sodann auch eingenommen wurden, und dergleichen). Die erkannten Bewegungen des Patienten können zur Auslösung von behandlungsbezogenen Funktionen und/oder Aktivitäten herangezogen werden, etwa nach einer Gesichts- oder Markierungserkennung, die eine Person im Erfassungsbereich als Patienten identifiziert bzw. klassifiziert, und Auswahl einer entsprechenden Anzahl dann zulässiger Parameter, Bewegungen, Funktionen und/oder Aktivitäten. Eine Echtzeitüberwachung des Dialyseplatzes ermöglicht eine Beobachtung des Patienten entfernt von dem Dialyseplatz, oder eine Bewegtbild-Gesprächsverbindung (Videotelefonie) mit dem Patienten. Außerdem sind Bedürfnisgänge des Patienten und dazu vorliegende oder fehlende Wiegevorgänge erkennbar bzw. der Vorgang "Wiegen vor und nach der Dialyse, sowie während der eigentlichen Dialysebehandlung", lückenlos per Video erfassbar und auswertbar. Teil des Grundgedankens ist auch, eine das Dialysegerät bedienende Person (Anwender) während ihrer Annäherung an das Gerät zu beobachten, zu unterstützen, Alarme des Geräts nach Erfassen geeigneter Gesten der Bedienperson stummzuschalten, und zumindest Teilfunktionen des Dialysegeräts, beispielsweise den Wechsel zwischen verschiedenen Bildschirmdarstellungen desselben, durch vorbestimmte Körperbewegungen, d. h. Gesten und/oder vorbestimmte Bewegungsabläufe bzw. Bewegungsmuster, zu steuern. Ebenfalls zum Grundgedanken gehört, bei Vorhandensein zumindest zweier Kameras eine Analyse der Blickrichtung des Bedieners bzw. Anwenders durchzuführen und vorzugsweise einen vom Bediener fixierten Bildschirmbereich hervorzuheben oder größer darzustellen, verschiedene Einstellparameter mittels Blickrichtungsanalyse und/oder Blickrichtungssteuerung auszuwählen, zu ändern, zu bestätigen und/oder zurückzunehmen, und interaktiv Hilfetexte darzustellen.

Es wird dazu eine Dialysevorrichtung mit einer Bewegungserkennungseinrichtung vorgeschlagen, bei der vernetzte, an der Dialysemaschine und/oder in deren Umgebung angeordnete und zur Erkennung und/oder Unterscheidung von Bewegungen in einem Erfassungsbereich ausgelegte Kamera- und/oder Sensoranordnungen bereitgestellt werden.

Insbesondere kann eine Umgebung einer Dialysemaschine mit mindestens einer Kamera beobachtet werden. Vorzugsweise ist die Kamera über ein Netzwerk mit einem ein Teil eines Dialysetherapiesystems bildenden Datenübernahme- und Auswertungssystem, das ein Überwachungssystem bildet, verbunden. Das Datenübernahme- und Auswertungssystem unterstützt ein Anzeigen und Speichern der gesamten Vorgänge als Video oder in Bildern. Das Datenübernahme- und Auswertungssystem kann eine automatisierte Auswertung dieser Aufnahmen unterstützen. Die Kamera kann in der Dialysemaschine eingebaut sein, in deren Umgebung mit Sicht auf für einen Behandlungsvorgang relevante Raumbereiche und/oder Geräteteile, wie beispielsweise eine Anzeige, oder an einem anderen dafür geeigneten Ort vorgesehen sein. Behandlungsvorgänge werden mit Bildern oder als Videoaufnahme(n) protokolliert und im Überwachungssystem gespeichert. Der Patient kann anhand einer Kombination aus Gewicht und Gesichtserkennung identifiziert werden. Mindestens kann sichergestellt werden, dass Patientenkarte und Patient zusammenpassen. Es kann erkannt werden, welcher Pfleger einen Wiegevorgang durchgeführt hat. Beim Wiegen **"nach Dialyse"** können auf einem Monitor Bilder des Wiegevorgangs **"vor Dialyse"** gezeigt werden, um Fehler zu vermeiden. Gibt es unerklärliche Gewichtsunterschiede zwischen vorher und nachher, können die Wiegevorgänge auf einem Monitor oder automatisch per Bildabgleich miteinander verglichen werden. Rollstühle und dergleichen können über Barcode oder Farbcodierung identifiziert werden, und eine Abweichung bzw. ein Offset kann automatisch herausgerechnet werden. Das Gewicht der Rollstühle kann dazu in einer Datenbank hinterlegt sein. Ein Start einer Wägung kann durch Gestensteuerung initiiert werden. Die Gestensteuerung kann sich auf den gesamten Ablauf des kameragestützt überwachten Wiegevorgangs oder Behandlungsvorgangs erstrecken

Ferner kann eine Dialysemaschine mit wenigstens einer daran oder an einem anderen geeigneten Ort angebrachten Kamera vorgesehen sein. Der Patient wird während des Dialysevorgangs mittels der Kamera beobachtet. Vorzugsweise ist die Kamera über ein Netzwerk mit einem Überwachungssystem verbunden. Der gesamte Vorgang kann dann im Überwachungssystem gespeichert werden. Programmroutinen analysieren aus gespeicherten Daten Bewegungen des Patienten und erkennen Nahrungsaufnahmen und/oder Flüssigkeitsaufnahmen und dergleichen. Eine Qualitätssicherung von Maßnahmen während der Dialyse kann durchgeführt werden, etwa in Bezug darauf, ob Medikamente verabreicht und auch eingenommen wurden usw. Das Bild der Kamera kann auch als eine Direktübertragung ("Online"- oder Live-Übertragung) genutzt werden, um nach dem Patienten zu sehen. Das Bild der Kamera kann darüber hinaus auch für eine Videotelefonie-Verbindung mit dem Patienten genutzt werden. Ferner kann das System etwa einen Gang des Patienten zu Waschräumen, Toiletten und dergleichen erkennen und ermitteln, ob ein entsprechender Wiegevorgang dazu durchgeführt wird oder wurde.

Insgesamt werden erfindungsgemäß Vorteile dahingehend erzielt, dass (nur) bei unerklärlichen Abweichungen des Gewichts vor und nach der Dialyse die Video-Aufzeichnungen ausgewertet werden (können), und dass mittels einer automatischen Auswertung der Videosequenzen relevante Szenen herausgefiltert werden (können) und es dem Personal ermöglicht wird, diese zu bewerten und ein gegebenenfalls vorliegendes Fehlverhalten des Patienten und/oder Personalfehler auszuwerten und zu korrigieren. Patienten mit regelmäßigen Abweichungen können im System gespeichert werden, so dass das System darauf basierend einen Hinweis geben kann, dass der Wiegevorgang nicht selbstständig vom Patienten bzw. nur unterstützt durch oder im Beisein von Personal durchgeführt werden darf. Außerdem ergeben sich Vorteile aus einer verbesserten Steuerbarkeit des Dialyse- und/oder Behandlungsvorgangs durch Erkennen von Bewegungen und/oder Gesten des Anwenders (Patient und/oder Behandlungspersonal) und Umsetzung dieser Bewegungen und/oder Gesten in eine Ausführung von Funktionen des Dialysegeräts, der Dialysemaschine und/oder des Dialysesystems einerseits, und die Nutzung dieser Bewegung zur Auslösung und Bereitstellung von Funktionen und/oder Diensten, etwa einem Schwesternruf oder Videoferngesprächen.

Dazu wird insgesamt eine Anordnung unter Verwendung von vernetzten, an einer Waage, an einem Patientenbett und/oder einer Dialysemaschine oder in einem Raumbereich mit Blick auf zu erfassende Information liefernde Abschnitte derselben angebrachten Kameras und/oder Sensoren zur verbesserten Erfassung von Bewegungen bzw. Gesten des Anwenders vor, während und nach dem Dialysevorgang vorgeschlagen.

Die Aufgabe wird somit gelöst durch eine Dialysemaschine für eine Dialysebehandlung, mit zumindest einer Bewegungserfassungseinrichtung, die dazu angeordnet ist, in einem räumlichen Erfassungsbereich eine Bewegung einer sich in dem Erfassungsbereich befindenden Person zu erfassen und Information über die erfasste Bewegung auszugeben; einer Steuereinheit zur Steuerung der Dialysemaschine; und einer Schnittstelleneinheit zur Anzeige von Betriebsinformation, zur Bedienung und/oder zur Netzwerkkommunikation der Dialysemaschine , wobei die Steuereinheit dazu angeordnet ist, auf der Grundlage der Information über die erfasste Bewegung Anweisungen zur Steuerung der Dialysemaschine zu erzeugen und an steuerbare Komponenten der Dialysemaschine auszugeben, und Daten betreffend eine Anzeige von Betriebsinformation, eine Bedienung und/oder eine Netzwerkkommunikation mit der Schnittstelleneinheit auszutauschen; und die Schnittstelleneinheit dazu angeordnet ist, auf der Grundlage der ausgetauschten Daten und in Übereinstimmung mit der Information über die erfasste Bewegung die Anweisungen zur Steuerung und/oder Regelung der Dialysemaschine visuell nachzuführen und/oder eine Netzwerkkommunikation durchzuführen. Weiter umfasst die Dialysemaschine eine Gesichtserkennungsfunktion und/oder Markierungserkennungsfunktion als eine Funktion zur Unterscheidung mehrerer sich in dem Erfassungsbereich befindender Personen, wobei die Dialysemaschine dazu angeordnet ist, mittels der Gesichtserkennungsfunktion und/oder der Markierungserkennungsfunktion eine sich in dem Erfassungsbereich befindende Person zu klassifizieren und der Person entsprechend ihrer Klassifikation zulässige Bewegungen und/oder zulässige Bedienvorgänge zuzuordnen.

Hierdurch wird vorteilhaft erreicht, dass die Dialysemaschine einerseits nur durch autorisierte Personen bedienbar ist. Andererseits ist auch eine Unterscheidung zwischen autorisierten Personen möglich, beispielsweise bezüglich von einer Person zulässig ausführbarer Funktionalität, oder bezüglich Protokollierung, Signatur und dergleichen. Im Rahmen einer Markierungserkennung können darüber hinaus statisch angebrachte Markierungen, beispielsweise an einem Zugang des Patienten, überwacht und bei Veränderungen wie etwa Bewegung oder Farbwechsel der Markierungen Aktionen ausgelöst, oder Zustandsmeldungen und/oder Alarme und dergleichen initiiert werden.

Bevorzugt ist die Bewegungserfassungseinrichtung eine 3D-Kameraanordnung zur Erfassung von Bildinformation in drei Dimensionen, die Strukturlicht mit einem vorbestimmten Lichtmuster in den räumlichen Erfassungsbereich ausstrahlt und wieder empfängt, auf der Grundlage eines Vergleichs und/oder einer Auswertung des ausgestrahlten Lichtmusters und des empfangenen Lichtmusters Tiefeninformation enthaltende Bildinformation ermittelt, und daraus die Information über die erfasste Bewegung erzeugt.

Für eine Erfassung komplexer Bewegungen oder Gesten ist aus Bildinformation oder Bildfolgen extrahierbare Tiefeninformation, d.h. Information, die eine Aussage über beispielsweise einen Ort oder einen Verlauf einer Bewegung in einer dritten (räumlichen) Dimension ermöglicht, vorteilhaft. Solche Tiefeninformation ist durch Vergleichen von Bildfolgen, welchen ein definiertes strukturelles Muster zugrunde liegt, und Erfassen und Auswerten von Abweichungen zwischen einem Muster eines ersten (ausgesendeten) Bilds und einem Muster eines zweiten (empfangenen) Bilds mittels beispielsweise Triangulation praktisch und kosteneffizient erzeugbar.

Weiter bevorzugt kann die Bewegungserfassungseinrichtung eine 2D-Kamera aufweisen, die dazu angeordnet ist, zweidimensionale 2D-Bildinformation zu erfassen, die als Bewegt- und/oder Standbild mittels Netzwerkkommunikation über ein Netzwerk an eine entfernte Stelle übertragbar ist.

Zur lediglichen Beobachtung oder für Videotelefonie wird keine Tiefeninformation benötigt. Eine zusätzlich vorgesehene 2D-Kamera kann daher dazu genutzt werden, während eines Behandlungsvorgangs dem Anwender Anleitung oder Anweisungen beispielsweise zur Gestensteuerung zu geben, und ihn bei deren Ausführung zu beobachten, zu trainieren oder zu korrigieren, während die 3D-Kameraanordnung ausgeführte Bewegungen erfasst und entsprechende Steuerungsfunktionen und/oder Aktionen ausgelöst werden. In anderen Worten lässt sich während eines Behandlungsvorgangs eine Standbild- und/oder Bewegtbild-Übertragung aufschalten, die unabhängig von Vorgängen im Behandlungsbereich eine Beobachtung und/oder Aufzeichnung dieser Vorgänge ermöglicht.

Weiter bevorzugt kann die Bewegungserfassungseinrichtung eine 2D-Kamera zur Erfassung von Bildinformation in zwei Dimensionen aufweisen, die dazu ausgelegt ist, die Information über die erfasste Bewegung zu erzeugen.

Eine 2D-Kamera kann bei einfachen Anforderungen an Genauigkeit und Komplexität von Bewegungen oder Gesten, d. h. in Fällen, in welchen keine Tiefeninformation benötigt wird, genügen. So kann eine 2D-Kamera mit Bewegungsauswertung für einen Patienten vorgesehen sein, der während der Behandlung in der Regel an einer definiert fokussierbaren Raumposition bzw. Tiefe im Raum auf dem Patientenbett liegt und dem während der Behandlung nur einige einfache Gesten für vorbestimmte Vorgänge wie etwa einen Schwesternruf (beispielsweise durch langsames Winken) oder Dringlichkeitsanforderungen (beispielsweise durch schnelles Winken) zur Verfügung stehen sollen, während gleichzeitig bzw. parallel dazu erweiterte Funktionalität nur dem Behandlungspersonal über die 3D-Erfassung zugänglich bleibt und der Patient diese nicht auslösen kann.

Auch bevorzugt kann die Bewegungserfassungseinrichtung eine Sensoreinrichtung sein, die dazu ausgelegt ist, in einem Erfassungsbereich der Sensoreinrichtung eine Bewegung zu erkennen.

Abhängig von jeweiligen Anforderungen sind auch Sensoren einsetzbar, deren Ausgabeinformation auf anderen Erfassungsgrundlagen als auf bildgebenden Kameraprinzipien beruht.

Vorzugsweise ist die erfasste Bewegung eine Geste einer sich in dem Erfassungsbereich befindenden Person.

Mittels vorbestimmten Gesten, Körper(teil)bewegungen und/oder Körper(teil)drehungen kann ein allgemeiner oder gerätespezifischer, größerer oder kleinerer und/oder erweiterbarer Befehlssatz vordefiniert und trainiert werden. Bewegungen, die als außerhalb dieses Befehlssatzes liegend erkannt werden, können ignoriert oder zur Auslösung von Aufmerksamkeit erzeugenden Meldungen und/oder Alarmen genutzt werden. In Verbindung mit einer Markierungs- und/oder Personenerkennung sind Untersätze eines Befehlssatzes und/oder Funktionen freigebbar oder sperrbar. Für einen Anwender mit höherer Qualifikation (Arzt) kann ein größerer Befehlsumfang freigegeben werden als für einen Anwender mit geringerer Qualifikation (Pflegepersonal, Assistenz). Führt ein Anwender in vorbestimmbarer Weise wiederholt fehlerhafte, nicht erkennbare oder dem Behandlungsverlauf bzw. dem Zustand des Dialysegeräts entsprechend falsche Gesten aus, kann ein weiterer Anwender zur Unterstützung herbeigerufen werden, oder kann die Gestensteuerung gesperrt und bis auf weiteres oder einen geeigneten Rücksetzvorgang ausschließlich eine Bedienung direkt am Gerät zugelassen werden. Die Vorteile der Gestenerkennung bei der Dialysebehandlung lassen sich auf diese Weise mit verbesserter Sicherheit und Behandlungsqualität für den Patienten verbinden.

In einer bevorzugten Ausführungsform ist die Bewegungserfassungseinrichtung in ein die Dialysemaschine umschließendes Gehäuse integriert.

Hierdurch wird ein kompaktes, beispielsweise mobiles, Dialysegerät bereitgestellt, welches eine Behandlung unmittelbar und ohne separat herzustellende Anschlussverbindungen betriebsfähig ist. Es versteht sich, dass auch in diesem Fall die Bewegungserfassungseinrichtung drehbar und/oder neigbar angelenkt sein kann, und dass das Dialysegerät zusätzliche Anschlüsse für weitere Bewegungserfassungseinrichtungen aufweisen kann.

In einer alternativ bevorzugten Ausführungsform kann dementsprechend zumindest eine Bewegungserfassungseinrichtung außerhalb eines die Dialysemaschine umschließenden Gehäuses angeordnet sein.

Hierdurch wird es vorteilhaft möglich, den Erfassungsbereich festzulegen, einzuschränken oder auszuweiten. Beispielsweise kann in Fällen, in welchen eine oder mehrere Dialysemaschine(n) in räumlich offener Umgebung mit entsprechendem Personenaufkommen betrieben werden, eine außerhalb der Dialysemaschine, beispielsweise an einem Patientenbett, in der Nähe des Zugangs des Patienten oder dergleichen erfassende Einrichtung auf für den jeweiligen Behandlungsvorgang wesentliche Erfassungsbereiche gerichtet werden, so dass sich Personen außerhalb dieser Erfassungsbereiche freier bewegen können und weniger oder keine Rechenlast und/oder weniger oder kein Datenaufkommen erzeugt wird. Schließlich erschließen außerhalb der Dialysemaschine angeordnete Erfassungseinrichtungen auch eine Erweiterbarkeit, eine Aufrüstung mit verbesserter oder bei Ausfall ersatzweiser Technologie, und eine größere Unabhängigkeit vom Aufstellungsort der Maschine selbst

Weiter bevorzugt ist die Dialysemaschine dazu angeordnet, mittels der Funktion zur Unterscheidung mehrerer sich in dem Erfassungsbereich befindender Personen der Person entsprechend ihrer Klassifikation eine in Bezug auf eine aus einer Bewegung ableitbare Dringlichkeit abgestufte Bedeutung einer Bewegung zuzuordnen. In anderen Worten ist es möglich, mittels der Gesichtserkennungsfunktion und/oder der Markierungserkennungsfunktion eine sich in dem Erfassungsbereich befindenden Person zu klassifizieren, und der Person entsprechend ihrer Klassifikation zulässige Bewegungen, zulässige Bedienvorgänge und/oder eine in Bezug auf eine aus einer Bewegung ableitbare Dringlichkeit abgestufte Bedeutung einer Bewegung zuzuordnen.

Hierdurch kann eine automatisierte oder vordefiniert hinterlegte Rechtevergabe und/oder Zuweisung von Prioritäten eingerichtet werden, die die Transparenz, die Kontrollierbarkeit, die Zuverlässigkeit und auch die Sicherheit des Behandlungsvorgangs und des Patienten verbessern.

Noch bevorzugt wird bei der Dialysemaschine eine von der Bewegungserfassungseinrichtung in dem Erfassungsbereich erkannte Bewegung derart auf eine berührungsempfindliche Anzeigeeinrichtung der Dialysemaschine gespiegelt, dass auf der Anzeigeeinrichtung eine mittels der Bewegung veranlasste und/oder ausgeführte Funktion der Dialysemaschine analog zu einer auf Berühren der Anzeigeeinrichtung erfolgenden Veranlassung und/oder Ausführung visuell nachgeführt, nachgebildet und/oder bestätigt wird.

Damit wird erreicht, dass bei Vorhandensein einer berührungsempfindlichen Anzeigeeinrichtung an der Dialysemaschine, beispielsweise einem Bildschirm (Touch Screen), über welchen die Maschine auch gestenlos vollständig steuerbar ist, die von der Bewegungserfassungseinrichtung erfassten Bewegungen bzw. die erfasste Gestik jederzeit analog abgebildet wird. In anderen Worten spiegelt die Anzeigeeinrichtung immer den aktuellen Behandlungszustand und den aktuellen Maschinenzustand wider, so dass alternativ zur Gestensteuerung jederzeit auch direkt durch Bedienung an der berührungsempfindlichen Anzeigeeinrichtung in den Behandlungsvorgang und/oder die Maschinensteuerung eingegriffen werden kann.

Die Erfindung wird nachstehend anhand bevorzugter Ausführungsbeispiele unter Bezugnahme auf die beigefügte Zeichnung näher beschrieben. Es zeigen:
Fig. 1 eine vereinfachte Darstellung eines vernetzten Dialysetherapiesystems mit einer vernetzten Patientenwaage und vernetzten Aufzeichnungskameras gemäß einem ersten Ausführungsbeispiel;
Fig. 2 eine vereinfachte Darstellung eines Ablaufs eines Wiegevorgangs eines Patienten mit automatischer Videoaufzeichnung gemäß dem ersten Ausführungsbeispiel für eine Dialysebehandlung;
Fig. 3 eine vereinfachte Darstellung eines Ablaufs eines Wiegevorgangs mit Videoaufzeichnung gemäß dem ersten Ausführungsbeispiel in Zusammenwirkung mit einem verbundenen Datenüberwachungs- und Auswertungssystem;
Fig. 4 eine vereinfachte Darstellung eines bekannten Ablaufs eines Wiegevorgangs unter Verwendung einer Protokollierung erfasster Daten auf Papier; und
Fig. 5 eine vereinfachte Blockdarstellung einer Dialysemaschine mit Bewegungserfassungseinrichtung und Gestensteuerung gemäß einem zweiten Ausführungsbeispiel.

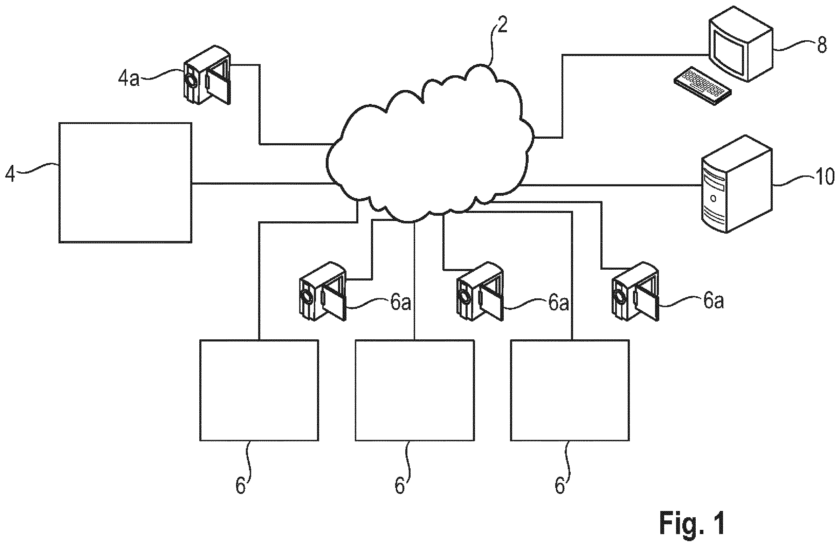

Fig. 1 zeigt eine vereinfachte Darstellung eines vorzugsweise vernetzten Gewichtsüberwachungssystems mit einer entsprechend vorzugsweise vernetzten Gewichtsüberwachungsvorrichtung gemäß einem ersten Ausführungsbeispiel. Das Gewichtsüberwachungssystem und die Gewichtsüberwachungsvorrichtung werden bevorzugt auf einer Dialysestation zur Dialysebehandlung von Patienten mit Niereninsuffizienz eingesetzt. Die verwendete Gewichtsüberwachungsvorrichtung ist hierbei bevorzugt eine Patientenwaage an sich bekannter Art, mittels der das Gewicht eines Dialysepatienten vor, während und nach der Dialysetherapie ermittelbar ist. Das Gewichtsüberwachungssystem bildet zumindest Teil eines bevorzugt vernetzten Gesamtsystems, in welches auch die Dialysemaschinen selbst, sowie verschiedenartige unterstützende Personal- bzw. Arztplätze mit Datenstationen und/oder angebundenen Servereinrichtungen bzw. Rechner oder Computer eingebunden sein können.

Im Einzelnen zeigt Fig. 1 beispielhaft aber nicht darauf beschränkend ein Netzwerk oder Datennetzwerk 2, über welches die verschiedenen in das Gesamtsystem eingebundenen Geräte und Einrichtungen miteinander verbunden sind. Das Netzwerk 2 ist nicht auf eine bestimmte Netzwerkart oder darüber vernetzte Geräteart beschränkt und kann beispielsweise ein an sich bekanntes lokales Netzwerk (LAN), ein drahtloses Netzwerk (WLAN), ein Mobilfunk-Netzwerk, eine Kombination aus diesen oder eine einfache Verkabelung sein. Ebenfalls ist die Art der über das Netzwerk 2 übertragbaren Daten nicht auf eine bestimmte Datenform beschränkt. Insbesondere sind aber Bild- und Videodaten sowie für die Dialysebehandlung benötigte Daten in wahlfreier Richtung über das Netzwerk 2 sende- und empfangbar. Zusätzlich kann das Netzwerk 2 verschiedene Schnittstellen und Übergänge bereitstellen, beispielsweise einen Zugang zum Internet, zu einem Intranet einer Einrichtung, in deren Zuordnungsbereich sich die Dialysestation befindet, oder zu einem Fernbereichsnetzwerk (WAN) anderer Art, etwa einer verschiedene Kliniken, Forschungseinrichtungen, Universitäten, Labore und dergleichen vernetzenden Struktur.

Zumindest eine Patientenwaage 4 bildet eine Gewichtsermittlungsvorrichtung oder eine Gewichtserfassungsvorrichtung in dem Netzwerk 2. Die grundlegende Ausführungsform und Wiegefunktionen der Patientenwaage 4 sind an sich bekannt und ebenfalls nicht auf eine bestimmte Form und/oder Funktion beschränkt.

Fig. 1 zeigt ferner zumindest eine Dialysemaschine 6, mit welcher die Dialysebehandlung eines Patienten durchführbar ist. Beispielhaft sind drei in dem Netzwerk 2 vernetzte Dialysemaschinen 6 dargestellt.

Außerdem können zumindest eine Arbeits- oder Datenstation 8 mit zumindest einer Tastatur und einem Bildschirm, wie beispielsweise ein Client-Computer oder ein Terminal, der oder das sich an einem Personal- oder Arztplatz befinden kann, und zumindest ein Server-Computer 10, auf dem eine Datenbank und/oder Software und Rechenleistung für die Arbeitsstation 8 bereitgestellt sein kann, in dem Netzwerk 2 vorgesehen und vernetzt sein.

Mit dem Bezugszeichen 4a ist zumindest eine erste Bilderfassungseinrichtung oder Bildinformationsaufnahmeeinrichtung, mithin zumindest eine erste Kamera, bezeichnet. Die Kamera 4a ist in diesem Ausführungsbeispiel eine vernetzbare Netzwerk-Videokamera, d. h. eine Kamera, die mit dem Netzwerk 2 verbunden ist und zur Aufnahme von Still- und/oder Bewegtbildern innerhalb eines Aufnahme- oder Erfassungsbereichs beispielsweise eines Objektivs oder Bildsensors der Kamera sowie zu deren Weiterleitung an andere Empfangs- und/oder Weiterverarbeitungseinrichtungen in dem Netzwerk 2 ausgelegt ist. Die Kamera 4a kann dabei zur Erzeugung und/oder Verarbeitung von analogen und/oder digitalen Bilddaten eingerichtet sein, bevorzugt werden jedoch digitale Bilddaten erzeugt und genutzt.

Die erste Kamera 4a kann entweder direkt an der Patientenwaage 4 oder getrennt von dieser in einem Teil des Raumes an einer vorbestimmten Position angeordnet sein. Die Kamera 4a ist dabei insbesondere so angeordnet, dass ihr Aufnahmebereich für die Dialysetherapie relevante Vorgänge an der Patientenwaage 4, wie beispielsweise einen Stand des Patienten auf der Waage, äußerlich erkennbare Merkmale des Patienten, wie beispielsweise dessen Kleidung und Schuhe, einen Umgebungsbereich der Patientenwaage 4, in welchem sich der Patient möglicherweise abstützen kann, von dieser angezeigte Daten wie beispielsweise das ermittelte Gewicht des Patienten, und/oder den Verlauf des Wiegevorgangs, beispielsweise ob und wie der Patient sich bewegt, und dergleichen umfasst und erfassen kann. In anderen Worten ist die zumindest eine Kamera 4a derart angeordnet und ausgerichtet, dass mit ihr die Patientenwaage 4 beobachtet werden kann.

Mit dem Bezugszeichen 6a ist zumindest eine zweite Bilderfassungseinrichtung oder Bildinformationsaufnahmeeinrichtung, mithin eine zweite Kamera, bezeichnet. Die zweite Kamera 6a ist in diesem Ausführungsbeispiel ebenfalls eine vernetzbare Netzwerk-Videokamera, d. h. eine Kamera, die mit dem Netzwerk 2 verbunden ist und zur Aufnahme von Still- und/oder Bewegtbildern innerhalb eines Aufnahme- oder Erfassungsbereichs beispielsweise eines Objektivs oder Bildsensors der Kamera sowie zu deren Weiterleitung an andere Empfangs- und/oder Weiterverarbeitungseinrichtungen in dem Netzwerk 2 ausgelegt ist. Die Kamera 6a kann dabei zur Verarbeitung von analogen und/oder digitalen Bilddaten eingerichtet sein, bevorzugt werden jedoch digitale Bilddaten genutzt.

Die Kamera 6a kann entweder direkt an der Dialysemaschine 6 oder getrennt von dieser in einem Teil des Raumes an einer vorbestimmten Position angeordnet sein. Es kann jeweils eine dedizierte Kamera 6a für jede von mehreren Dialysemaschinen 6 oder eine Kamera 6a für mehrere Dialysemaschinen 6 vorgesehen sein. Die Kamera 6a ist dabei insbesondere so angeordnet, dass ihr Aufnahmebereich für die Dialysetherapie relevante Vorgänge an der Dialysemaschine 6, wie beispielsweise einen Zustand oder ein Verhalten eines an die Maschine angeschlossenen Patienten, äußerlich erkennbare Merkmale des Patienten, wie beispielsweise dessen Kleidung und Schuhe, einen Umgebungsbereich der Dialysemaschine 6, von dieser angezeigte Daten, und/oder den Verlauf des Dialysevorgangs und dergleichen umfasst und erfassen kann. In anderen Worten ist die zumindest eine zweite Kamera 6a derart angeordnet und ausgerichtet, dass mit ihr eine oder mehrere der Dialysemaschinen 6 beobachtet werden können.

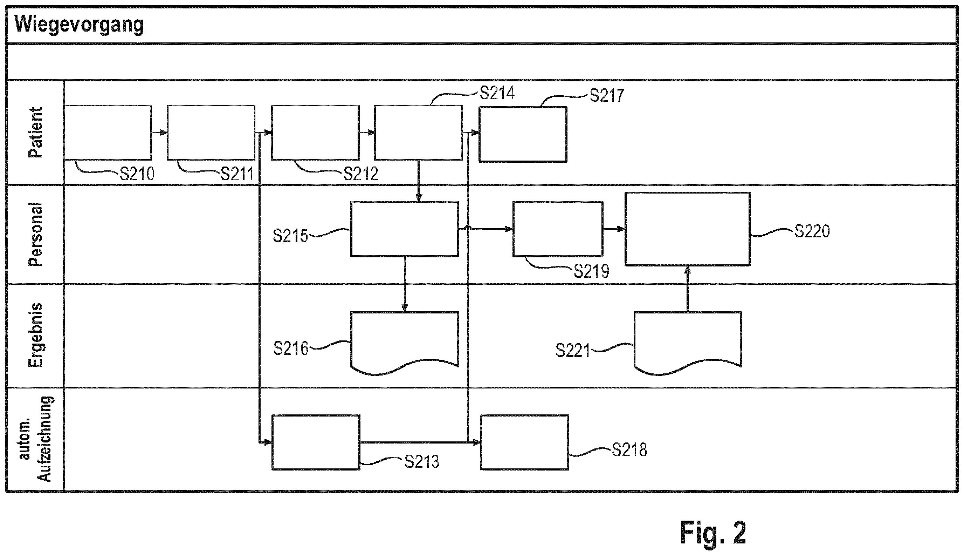

Nachstehend wird ein zeitlicher Ablauf eines Wiegevorgangs mit einer automatischen Bild- und/oder Videoaufzeichnung gemäß einem Ausführungsbeispiel für eine Dialysebehandlung näher beschrieben. Fig. 2 zeigt, auszugsweise anhand von vor der Dialysebehandlung durchzuführenden Schritten bzw. Aktivitäten, eine vereinfachte Darstellung eines solchen Ablaufs. Auszugsweise bedeutet, dass sich entsprechende Schritte und Aktivitäten auch während der auf den Vorbereitungsabschnitt folgenden Dialysebehandlung und auch nach deren Ende anschließen können.

Es wird angemerkt, dass der in Fig. 2 gezeigte Wiegevorgang vollständig manuell gesteuert werden kann, alternativ aber auch zumindest teilweise automatisiert ablaufen kann. Insbesondere kann hierbei die automatische Bild- und/oder Videoaufzeichnung manuell oder ereignisgesteuert, beispielsweise basierend auf Signalisierungen der Patientenwaage 4 oder auf Mechanismen einer Gestenerkennung, zum Beispiel Deut- und/oder Winkbewegungen vorbestimmter Art und/oder Richtung, in Gang gesetzt und/oder beendet werden, während etwa die Aufnahme sowie eine Übertragung und Weiterleitung erzeugter Bild- und/oder Videodaten über das Netzwerk 2 an eine nachgeordnete Weiterverarbeitungseinrichtung, z.B. den Server 10 oder die Arbeitsstation 8, und eine dortige Aufbereitung der Daten automatisiert erfolgen können.

In dem in Fig. 2 gezeigten Ablauf sind in chronologischer Abfolge und ausschnittsweise Schritte oder Aktivitäten angegeben, die während des Wiegevorgangs eines Patienten, beispielsweise eines Dialysepatienten mit Niereninsuffizienz, im Rahmen eines Therapieablaufs mit vorbereitendem Wiegen vor der Therapie von dem Patienten und Personal einer in diesem Beispielfall Dialysestation durchzuführen sind. Insoweit Aktivitäten und/oder Schritte parallel und/oder gleichzeitig durchzuführen sind, ist die Nummerierung der einzelnen Schritte nicht zwingend als Abfolge oder Aufeinanderfolge zu verstehen, sondern kann aus Zweckmäßigkeitsgründen auch nur eine Angabe von Bezugszeichen zur eindeutigen Zuordnung darstellen.

In einem Schritt S210 beginnt der Vorbereitungsablauf damit, dass beispielsweise der Patient in der Dialysestation eintrifft und für das Wiegen vorbereitende Maßnahmen wie etwa ein vorbestimmtes Ablegen von Kleidung, Schuhen, Gepäck und dergleichen durchführt. In einem Schritt S211 betritt der Patient sodann die Patientenwaage 4, oder nimmt auf ihr Platz. Gegebenenfalls kann dieser Vorgang von Personal der Dialysestation helfend unterstützt werden. Nachdem sich der Patient auf der Patientenwaage 4 befindet, wird der Wiegevorgang eingeleitet. Dies kann durch den Patienten selbst, durch das Personal, oder automatisch erfolgen. Automatisch beispielsweise dadurch, dass die für die Patientenwaage 4 zuständige erste Kamera 4a nach Art eines Direkt- oder Live-Bilds den Patienten auf der Patientenwaage 4 bereits erfasst, ohne bereits Bilddaten aufzuzeichnen, und auf die Erfassung beispielsweise einer als Start- oder Bereit-Signal interpretierbaren, vorbestimmten Geste des Patienten oder des Personals hin den Wiegevorgang und/oder dessen Aufzeichnung in Gang setzt. Möglichst zeitgleich mit dem in Gang setzen des Wiegevorgangs wird in einem Schritt S213 eine Aufzeichnung der von der Kamera 4a erzeugten und gelieferten Bildsignale und/oder Bilddaten begonnen. Eine solche Aufzeichnung dauert vorzugsweise solange ununterbrochen an, bis ein definiertes Ende des Wiegevorgangs signalisiert und/oder erfasst wird. Während die Aufzeichnung des Wiegevorgangs andauert, wird von dem Patienten in einem Schritt S212 erwartet, dass er während des Wiegens im Hinblick auf ein verlässliches und genaues Wiegeergebnis zumindest für eine vorbestimmte Zeitspanne stillsteht. Nach Ablauf des eigentlichen Wiegens des Patienten, welches beispielsweise durch die Patientenwaage 4 nach Verstreichen einer vorbestimmten, ausreichenden Zeitspanne signalisiert werden kann, wird in einem Schritt S214 das ermittelte oder festgestellte Gewicht an der Waage ausgelesen oder abgelesen und in einem Schritt S215 von dem Personal dokumentiert, beispielsweise in einem Schritt S216 in einem Protokoll festgehalten. In einem Schritt S218 wird sodann die Aufzeichnung durch die Kamera 4a angehalten oder beendet, und danach kann in einem Schritt S217 der Patient die Patientenwaage 4 wieder verlassen. Sodann ermittelt in einem Schritt S219 das Personal aus dem in Schritt S215 dokumentierten Gewicht das Ultrafiltrationsvolumen bzw. die Ultafiltrationsmenge und überträgt diese(s) in einem Schritt S220 als einen von Behandlungsparametern für die Dialysebehandlung in das Dialysegerät 6. Erforderliche Behandlungsparameter können von dem Personal in einem Schritt S221 auch aus dem Protokoll entnommen oder aus diesem abgelesen werden.

Dadurch, dass der Wiegevorgang an der Patientenwaage 4 durch den Aufzeichnungsvorgang mittels der Kamera4a überwacht, aufgezeichnet und für eine spätere Wiedergabe und/oder Weiterverarbeitung abgespeichert wird, wird der gesamte relevante Wiegevorgang über einen zweiten Protokollweg unabhängig von den Aktivitäten des Personals zusätzlich dokumentiert. Werden zu einem Zeitpunkt Abweichungen oder Unstimmigkeiten festgestellt, die einer Überprüfung bedürfen, kann auf die durch die Aufzeichnung bereitgestellte zusätzliche Dokumentation zugegriffen werden.

Beispielsweise kann die Kamera so ausgerichtet sein, dass ein Bild oder eine Aufzeichnung des Patienten zur Verfügung gestellt wird, während er die Waage betritt. Anhand der Aufzeichnung wird nachvollziehbar oder überprüfbar, ob der Patient vorschriftsmäßig Kleidung, Jacke, Schuhe und dergleichen abgelegt hatte, bevor der Wiegevorgang begonnen hat. Mehrere zeitlich beabstandete Aufzeichnungen können beispielsweise Aufschluss darüber liefern, ob zwei in Zusammenhang stehende oder in Zusammenhang zu bringende Wiegevorgänge unter unterschiedlichen Voraussetzungen stattgefunden haben. Falls beispielsweise das Tragen von Schuhen während des Wiegevorgangs zulässig ist, kann festgestellt werden, ob ein Wiegevorgang mit schweren Schuhen und ein anderer Wiegevorgang mit leichten Schuhen durchgeführt wurde.

Ist die Aufzeichnung der Kamera mit einem Datums- und/oder Zeitstempel versehen, können Abweichungen, die auf die Tageszeit zurückzuführen sind, wie beispielsweise ein Wiegevorgang am Morgen oder ein Wiegevorgang am Abend, ermittelt werden.

Ferner können anhand der Aufzeichnung mittels der Kamera 4a Ablesefehler durch das Personal ermittelt werden. Ist die Kamera so ausgerichtet, dass eine Anzeige, beispielsweise eine Gewichtsanzeige in Kilogramm auf einer Anzeigevorrichtung der Patientenwaage 4 oder ein Ausschlag oder ein Gleichgewichtszustand an einer Skala der Patientenwaage aufgezeichnet wird, kann im Nachhinein festgestellt werden, ob durch das Personal bei der Dokumentation des Patientengewichts in Schritt S215 korrekt abgelesen und/oder eine Ablesung in Schritt S216 korrekt in das Protokoll übertragen wurde. Wird zusätzlich eine Aufzeichnung des Protokolls, etwa als Videosequenz oder Standbild, angefertigt, kann ferner in Schritt S220 geprüft werden, ob ein aus dem Protokoll entnommener und in die Dialysemaschine 6 übertragener Behandlungsparameter korrekt abgelesen wurde.

Um derartige unterschiedliche Aspekte des Wiegevorgangs in eine Aufzeichnung einzubinden, kann insbesondere vorgesehen sein, dass die Kamera 4a bzw. ein Aufnahmeabschnitt derselben derart manuell und/oder motorisch schwenkbar und/oder zwischen einer Naheinstellung und einer Ferneinstellung oder Teleeinstellung verfahrbar ist. Dazu kann vorgesehen sein, dass die Kamera 4a entsprechend einem Zeitverlauf des Wiegevorgangs zu diesem korrespondierend nachgeführt wird, etwa durch Programmroutinen einer Kamerasteuerung, die auf eine Gestensteuerung ansprechen, nach Art eines Schaltwerks automatisch fortschreitend und/oder wechselnd, oder auf der Grundlage von Befehlen, die der Kamera über das Netzwerk 2 zugeführt werden.

Bei einer manuellen Zustandsänderung der Kamera 4a ist ferner denkbar, den Wiegevorgang in vorbestimmte Abschnitte einzuteilen, wie beispielsweise "Wiegebeginn", "Ablesung" und dergleichen, und die Patientenwaage 4 so mit der Kamera 4a zu koppeln, dass der Wiegevorgang nach Erreichen des Endes eines Abschnittes erst dann fortgeführt werden kann, nachdem die Kamera 4a auf eine neue Ausrichtung oder Einstellung geändert wurde. Beispielsweise kann dabei vorgesehen sein, dass die Patientenwaage 4 einen ermittelten und auszugebenden Wert erst dann zur Anzeige bringt, wenn sie ein Bestätigungssignal oder eine bestätigende Aktivität des Personals dahingehend erfasst hat, dass die Kamera 4a zur Aufzeichnung der Anzeige bzw. des angezeigten Werts ausgerichtet wurde und bereit ist.

Insoweit erfolgt gemäß Fig. 2 grundlegend eine Aufzeichnung von zumindest für eine Dokumentation, Nachprüfung, Nachvollziehbarkeit und, da die Aufzeichnung des Wiegevorgangs vorzugsweise vor der Übertragung von Behandlungsparametern in die Dialysemaschine 6 angehalten wird, gegebenenfalls Korrektur solcher Behandlungsparameter relevanter Abschnitte des Wiegevorgangs eines Patienten auf der Patientenwaage 4. Gegebenenfalls kann aber die Übertragung der Behandlungsparameter ebenfalls mit aufgezeichnet werden. Hierzu kann beispielsweise die Aufzeichnung nach Schritt S218 erneut gestartet und nach Schritt S220 erneut beendet werden, falls der Aufnahmebereich der Kamera 4a auch eine Behandlungsparameter-Eingabeumgebung umfasst oder die Kamera 4a zuvor entsprechend ausgerichtet wurde. Alternativ kann die Übertragung der Behandlungsparameter in die Dialysemaschine 6 auch von der dort vorgesehenen Kamera 6a aufgezeichnet werden.

Ein Speicherort der Aufzeichnung der Kamera 4a ist nicht auf einen bestimmten Speicherort beschränkt. Beispielsweise kann die Speicherung der Aufzeichnung lokal in der Kamera 4a selbst, etwa auf einem mit der Kamera 4a verbundenen Datenträger wie beispielsweise einer wechselbaren Speicherkarte, einem internen Datenträger oder einem von extern verbindbaren Datenträger erfolgen. Eine Kopie oder Sicherungskopie der Aufzeichnung kann jederzeit über das Netzwerk 2 an einem zusätzlichen Speicherort erstellt werden. Die Speicherung der Aufzeichnung kann alternativ auch unmittelbar durch Übertragung der erzeugten Bild- und/oder Videodaten über das Netzwerk 2 an eine vorbestimmte Speichereinrichtung erfolgen, etwa bei Verwendung einer speicherlosen Kamera 4a. Alternativ kann eine Speicherung der Aufzeichnung auch in einer entsprechend dazu ausgelegten und mit einer Speichereinrichtung und/oder einer zur Wiedergabe der Aufzeichnung eingerichteten Anzeigeeinrichtung versehenen Patientenwaage 4 erfolgen. Vorteilhaft ist hierbei, dass ein Wiegevorgang und dessen Dokumentation in Bild und Video an Ort und Stelle verfügbar sind, so dass bedarfsweise eine sehr schnelle Kontrolle erfolgen kann, während sich zum Beispiel der Patient noch in der Nähe der Waage befindet und sich Wiegebedingungen etwa durch eine zwischenzeitliche Nahrungsaufnahme und dergleichen nicht wesentlich geändert haben, und gegebenenfalls ein Wiegevorgang sehr schnell wiederholt werden kann.

Nachstehend wird ein zeitlicher Ablauf eines Wiegevorgangs mit einer automatischen Bild- und/oder Videoaufzeichnung gemäß dem Ausführungsbeispiel für eine Dialysebehandlung in Zusammenwirkung mit einem verbundenen Datenübernahme- und Auswertungssystem näher beschrieben.

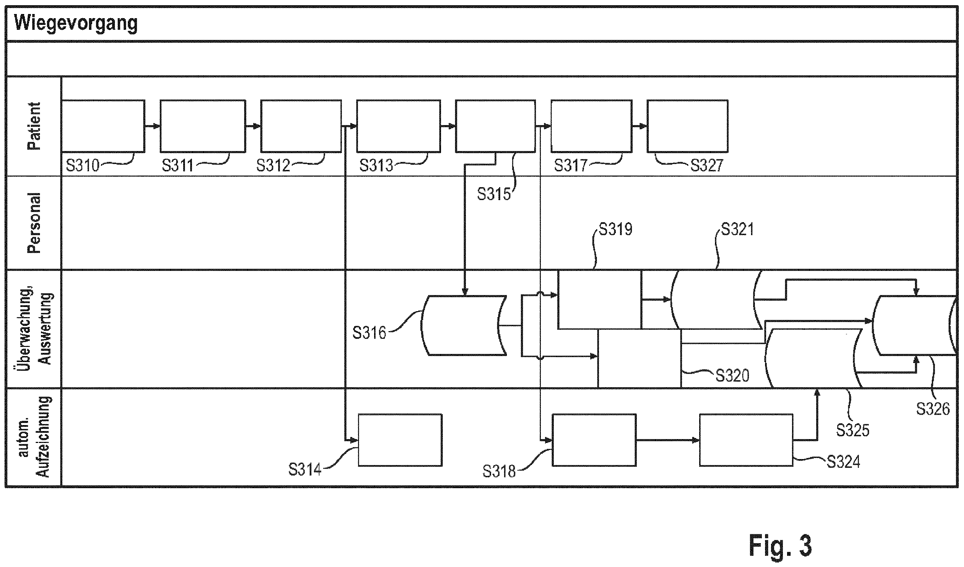

Fig. 3 zeigt, auszugsweise anhand von vor der Dialysebehandlung durchzuführenden Schritten bzw. Aktivitäten, eine vereinfachte Darstellung eines solchen Ablaufs. Auszugsweise bedeutet, dass sich entsprechende Schritte und Aktivitäten auch während der auf den Vorbereitungsabschnitt folgenden Dialysebehandlung und auch nach deren Ende anschließen können.

Es wird angemerkt, dass der in Fig. 3 gezeigte Ablauf und Wiegevorgang vollständig manuell gesteuert werden kann, alternativ aber auch teilautomatisiert bis hin zu vollautomatisiert ablaufen kann. Insbesondere kann die automatische Bild- und/oder Videoaufzeichnung manuell oder ereignisgesteuert, beispielsweise basierend auf Signalisierungen der Patientenwaage 4 oder auf Mechanismen einer Gestenerkennung, in Gang gesetzt und/oder beendet werden, während etwa die Aufnahme sowie eine Übertragung und Weiterleitung erzeugter Bild- und/oder Videodaten über das Netzwerk 2 an eine nachgeordnete Weiterverarbeitungseinrichtung, z.B. den Server 10 oder die Arbeitsstation 8, und eine dortige Aufbereitung der Daten automatisiert erfolgen können.

In dem in Fig. 3 gezeigten Ablauf sind in chronologischer Abfolge und ausschnittsweise Schritte oder Aktivitäten angegeben, die während des Wiegevorgangs eines Patienten, beispielsweise eines Dialysepatienten mit Niereninsuffizienz, im Rahmen eines Therapieablaufs mit vorbereitendem Wiegen vor der Therapie von dem Patienten und Personal einer in diesem Beispielfall Dialysestation durchzuführen sind. Insoweit Aktivitäten und/oder Schritte parallel und/oder gleichzeitig durchzuführen sind, ist die Nummerierung der einzelnen Schritte nicht zwingend als Abfolge oder Aufeinanderfolge zu verstehen, sondern kann aus Zweckmäßigkeitsgründen auch nur eine Angabe von Bezugszeichen zur eindeutigen Zuordnung darstellen.

Das in Fig. 3 insbesondere mitwirkende Datenübernahme- und Auswertungssystem kann hierbei eine über das Netzwerk 2 verbundene Software und Datenbank zur transparenten Abbildung und Steuerung von Vorgängen einer Dialysebehandlung sein. Ein grundlegende Ansätze und Funktionen bietendes System der Anmelderin ist bereits aus dem Stand der Technik hinlänglich bekannt und kann beispielsweise an der Arbeitsstation 8 und/oder dem Server 10 installiert oder über diese anderweitig zugänglich sein.

Im Wesentlichen übernimmt ein solches Datenübernahme- und Auswertungssystem Daten von mit ihm verbundenen Geräten, wie beispielsweise von der Patientenwaage 4 ermittelte Gewichte, von (nicht gezeigten) Analysegeräten während eines Überwachungs- bzw. Monitoring-Betriebsablaufs, und betreibt darüber hinaus eine Datenbank, über welche Zugriff auf Daten und Einträge von beispielsweise Krankenhausinformationssystemen, Laboren, Verwaltungen, externen Kliniken und Ärzte und dergleichen besteht. Ein in Abhängigkeit einer jeweiligen Funktionalität unidirektionaler oder bidirektionaler Datenaustausch zwischen angeschlossenen Geräten, der Datenübernahme/Datenerfassung/Datenüberwachung, der Datenbank, Arbeitsstationen oder Eingabeeinrichtungen und Lieferanten und/oder Benutzern der Daten der Datenbank ist möglich.

Fig. 3 stellt in einer Zeile "Überwachung, Auswertung" grundlegende Funktionen und Aufgaben dar, die gemäß dem Ausführungsbeispiel von dem Datenübernahme- und Auswertungssystem nach der Übernahme von Daten aus der Patientenwaage 4 in einem Schritt S316 und der Aufzeichnung und Aufbereitung des Wiegevorgangs in Schritten S314, S318 und S324 übernommen werden. Der Wiegevorgang und die Aufzeichnung werden dabei wie vorstehend unter Bezugnahme auf Fig. 2 beschrieben durchgeführt. Auch die Funktionen der Kamera 4a sind bis auf nachstehend dargestellte Unterschiede wie unter Bezugnahme auf Fig. 2 beschrieben.

Im Einzelnen beginnt der Ablauf nach Fig. 3 in einem Schritt S310. In einem Schritt S311 identifiziert sich der Patient an der Patientenwaage 4, beispielsweise mittels einer Chipkarte, einer Patientenkarte oder dergleichen, die von ihm oder von Personal in eine Kartenlesevorrichtung eingeführt wird. Das Datenübernahme- und Auswertungssystem erkennt den Patienten anhand dessen Identifikation und stellt gegebenenfalls bereits vorhandene Patientendaten aus der Datenbank bereit, oder kann einen neuen Patienteneintrag unter Verwendung der auf der Karte gespeicherten Daten anlegen. In einem Schritt S312 betritt der Patient die Patientenwaage 4 bzw. nimmt auf ihr Platz. Der Schritt S312 entspricht hierbei dem Schritt S211 in Fig. 2. In einem Schritt S313, der dem Schritt S212 in Fig. 2 entspricht, und einem Schritt S314, der dem Schritt S213 in Fig. 2 entspricht, wird der Wiegevorgang durchgeführt und dessen Aufzeichnung begonnen. In einem Schritt S315 wird anders als in Fig. 2 das von der Patientenwaage 4 gemessene oder ermittelte Gewicht, in diesem Ausführungsbeispiel das Ist-Gewicht, des Patienten nicht an der Patientenwaage 4 abgelesen, sondern an das Datenübernahme- und Auswertungssystem übertragen. Natürlich kann nach wie vor eine zusätzliche und ablesbare Anzeige des Gewichts an der Patientenwaage 4 vorgesehen sein. Nach Fig. 3 wird das Gewicht aber unmittelbar in das System übernommen und kann über dieses, vorzugsweise in zumindest nahezu Echtzeit, an einem Bildschirm dargestellt und abgelesen werden. Durch die direkte Übernahme in das System erfolgen auch unmittelbar eine sofortige Dokumentation des Gewichts und eine entsprechende Protokollierung, so dass in Fig. 3 die entsprechenden Schritte der Fig. 2 entfallen können. In einem Schritt S317, der dem Schritt S217 in Fig. 2 entspricht, verlässt der Patient die Patientenwaage 4 und entnimmt in einem Schritt S327 seine ihn identifizierende Karte bzw. lässt diese durch Personal entnehmen, und in einem Schritt S318, der dem Schritt S218 in Fig. 2 entspricht, wird die Aufzeichnung des Wiegevorgangs beendet.

Nach dem Schritt S316, in dem das durch den Wiegevorgang ermittelte Ist-Gewicht des Patienten vorliegt, verzweigt das Datenübernahme- und Auswertungssystem dessen Weiterverarbeitung in einen aus Schritten S319 und S321 bestehenden ersten Verarbeitungszweig und in einen aus einem Schritt S320 bestehenden zweiten Verarbeitungszweig.

In Schritt S319 wird ausgehend von dem in Schritt S316 erhaltenen Ist-Gewicht des Patienten ein Ultrafiltrationsvolumen bzw. eine Ultrafiltrationsmenge vor der Dialyse kalkuliert oder berechnet. In Schritt S321 wird diese berechnete Ultrafiltrationsmenge als Datensatz für die Dialysebehandlung, die hier nicht weiter dargestellt ist, zur Verfügung gestellt.

In Schritt S320 führt das Datenübernahme- und Auswertungssystem nach dem Ende der Dialysebehandlung eine Überprüfung der Ultrafiltrationsmenge durch. Die tatsächlich benötigte Ultrafiltrationsmenge ist nach der Dialysebehandlung bekannt und kann mit der vor der Dialysebehandlung anhand des Ist-Gewichts des Patienten errechneten Ultrafiltrationsmenge verglichen werden. Nicht vernachlässigbare Abweichungen können so unmittelbar festgestellt werden.

Möglichst zeitgleich oder parallel zu den Schritten S319 bis S321 wird in einem Schritt S324 nach dem Anhalten der Aufzeichnung in Schritt S318 die aufgezeichnete Bild- und/oder Videosequenz geeignet aufbereitet, beispielsweise zeitgestempelt, formatgewandelt, komprimiert, und/oder in von dem Datenübernahme- und Auswertungssystem verwendbare Videodatensequenzen und Standbild- bzw. Fotosequenzen des Wiegevorgangs oder vorbestimmter Abschnitte desselben unterteilt. In einem Schritt S325 übernimmt sodann das Datenübernahme- und Auswertungssystem die in Schritt S324 aufbereitete Bild- und/oder Videosequenz.

Sodann erzeugt das Datenübernahme- und Auswertungssystem in einem Schritt S326 ein Behandlungsprotokoll der vorangehenden Dialysebehandlung. In das Behandlungsprotokoll in Schritt S326 gehen zumindest der die vorausberechnete Ultrafiltrationsmenge enthaltende Datensatz aus Schritt S321, die Überprüfung der Ultrafiltrationsmenge nach der Dialysebehandlung gemäß Schritt S320, beispielsweise als Ergebnis eines Vergleichs oder als ein absoluter Wert, und die Videodaten und/oder Bild- bzw. Fotosequenz des Wiegevorgangs ein. Dabei kann beispielsweise in das Behandlungsprotokoll eine Verknüpfung zu den andernorts gespeicherten Bild- und Videosequenzen eingetragen werden, über welche die Aufzeichnung des Wiegevorgangs erforderlichenfalls aufrufbar ist.

In Zuordnung zu dem eindeutig identifizierten Patienten sind insbesondere über mehrere Behandlungen, d. h. Tage oder Wochen, hinweg erstellte Aufzeichnungen entsprechender Wiegevorgänge bereitstellbar. Durch das Aufzeichnen der Wiegevorgänge mittels der Kamera 4a werden somit die einzelnen Abläufe auch längerfristig nachvollziehbar.

Hierzu kann vorgesehen sein, dass das Datenübernahme- und Überwachungssystem Bild- und Videodaten in Form wenigstens zweier nebeneinander angeordneter Fenster, die beispielsweise jeweils Standbilder oder Videosequenzen anzeigen, darstellt, wobei in jedem Fenster unabhängig voneinander Daten ein und desselben Wiegevorgangs oder zweier zeitlich auseinander liegender Wiegevorgänge anzeigbar sind. Somit kann auch dann, wenn das System bereits bei einem ersten Wiegevorgang eine Abweichung des Ist-Gewichts von einem Ist-Gewicht vergangener Zeiträume erfasst, der Wiegevorgang kontrolliert werden. Auf diese Weise können Ursachen wie beispielsweise das Tragen schwerer Schuhe anstelle von leichten Schuhen, eine nicht abgelegte Jacke, eine mitgewogene Tasche, ein Abstützen während des Wiegens und dergleichen erkannt werden. Treten ferner Abweichungen beispielsweise erst nach einem zweiten Wiegevorgang auf, kann gegebenenfalls eine Befragung des Patienten entfallen, da die Wiegevorgänge vermittels des Systems auch ohne eine solche Befragung nachvollziehbar sind. Als Ursachen für Abweichungen erst nach einem zweiten Wiegen können beispielsweise unterschiedliche Kleidung während beider Wiegevorgänge, unterschiedliche Schuhe während beider Wiegevorgänge, oder Gepäck bei einem oder beiden Wiegevorgängen erkannt werden.

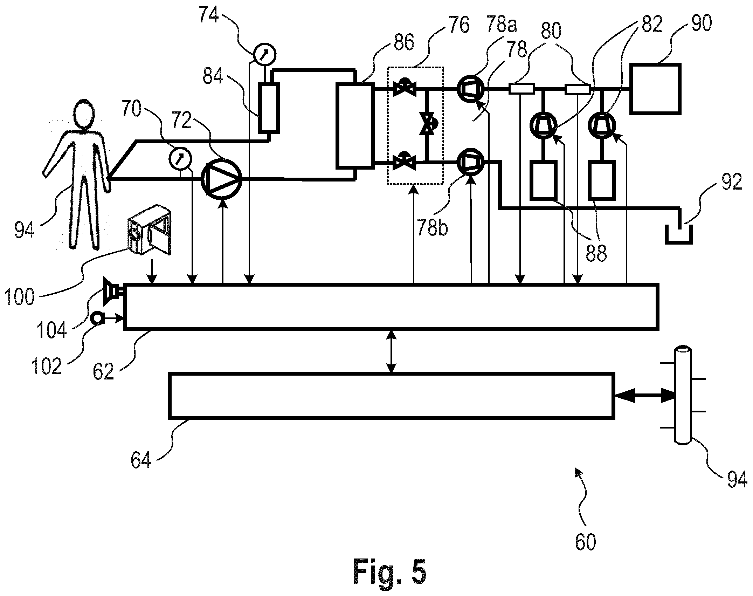

Fig. 5 zeigt eine vereinfachte Blockdarstellung einer für Gestensteuerung ausgelegten Dialysemaschine gemäß einem zweiten Ausführungsbeispiel. Die Dialysemaschine kann eine Dialysemaschine 6 mit einer Kamera 6a innerhalb des vernetzten Dialysetherapiesystems mit einer vernetzten Patientenwaage und vernetzten Aufzeichnungskameras gemäß dem ersten Ausführungsbeispiel sein, und dem ersten Ausführungsbeispiel entsprechende Funktionalität in entsprechender Kombination bereitstellen, oder eine Dialysemaschine, die in einer anderweitigen Behandlungsumgebung einsetzbar ist. Insoweit besteht keine Beschränkung der Dialysemaschine auf eine Verwendung in Verbindung mit der in dem ersten Ausführungsbeispiel beschriebenen Gewichtserfassung.

Fig. 5 zeigt Einzelnen eine Dialysemaschine 60 mit einer Steuereinheit 62, einer Schnittstelleneinheit 64, die eine Anzeige-, Bedien- und Kommunikationseinheit bildet, einer Bewegungserfassungseinrichtung 100, sowie dem Dialysevorgang zugeordneten, noch zu beschreibenden internen Komponenten der Dialysemaschine 60.

Die Schnittstelleneinheit 64 ist zum einen für eine bidirektionale Kommunikation und/oder Signalisierung mit der Steuereinheit 62 und andererseits mit einem Netzwerk 66, beispielsweise einem LAN und/oder WAN, verbunden.

Die Steuereinheit 62 ist ferner signalisierend, steuernd und/oder regelnd mit internen Komponenten der Dialysemaschine 60 für die Dialysebehandlung verbunden, beispielsweise unter anderem mit einem arteriellen Druckaufnehmer bzw. Aufnehmer für arteriellen Druck 70, einer Blutpumpe 72, einem venösen Druckaufnehmer bzw. Aufnehmer für venösen Druck 74, zumindest einem Bypassventil 76, einem Zulaufabschnitt 78a und einem Auslaufabschnitt 78b einer Flusspumpe 78, zumindest einer Leitfähigkeitssonde 80, und zumindest einer Konzentratpumpe 82. Die Steuereinheit 62 empfängt Erfassungssignale von den Aufnehmern und der zumindest einen Sonde 70, 72 und 80, und gibt Steuer- und/oder Regelsignale an die Pumpen 72, 78 und 82 aus.

Innerhalb eines Flüssigkeitsleitungssystems der Dialysemaschine 60 sind darüber hinaus eine venöse Kammer 84, ein Dialysator 86, zumindest ein Konzentratbehälter 88, eine Aufnahme für Dialysewasser 90, und ein Abflussabschnitt 92 vorgesehen. Vorwiegend wird das Flüssigkeitsleitungssystem über eine Schlauchanordnung mit dem Körper eines Patienten verbunden. Insoweit sind Aufbau und Funktionsweise der Dialysemaschine 60 an sich bekannt, eine nähere Beschreibung derselben wird daher an dieser Stelle weggelassen.

In diesem Ausführungsbeispiel sind ferner die Bewegungserfassungseinrichtung 100, beispielsweise eine Kameraanordnung und/oder eine Sensoranordnung einschließlich zumindest einer 3D-Kamera, zumindest einer 2D-Kamera und/oder einem kameralosen Sensor, der eine Bewegung anders als bildbasiert erfasst, sowie zumindest ein Mikrofon 102 und zumindest ein Lautsprecher 104 mit der Steuereinheit 62 verbunden. Das Mikrofon 102 und der Lautsprecher 104 können in die Bewegungserfassungseinrichtung 100 integriert oder separat von dieser angeordnet sein.

Die Bewegungserfassungseinrichtung 100 ist bevorzugt eine 3D-Kamera, welche eine Beobachtung, Erfassung und/oder Aufnahme eines Orts oder Raums in drei Dimensionen erlaubt, diese Beobachtung, Erfassung und/oder Aufnahme in einen synchronisierten Bildstrom mit Tiefeninformation und Farbinformation umsetzt, und sodann die synchronisierten Bilder weiter in beispielsweise Identifikationsinformation bezüglich Menschen, wie körperliche Eigenschaften, Bewegungen, Gesten und dergleichen, Klassifizierungsinformation bezüglich Objekten und Gegenständen, und/oder Ortung von etwa Wänden und Böden umsetzt.

Die Bewegungserfassungseinrichtung 100 bildet insgesamt eine Hardware-Komponente zur unter anderem Erfassung von Gesten und Bewegungen, die vorzugsweise einen Strukturlichtsender in Form beispielsweise eines Infrarotlicht-Senders (IR-Laser mit beispielsweise einer Klasse 1-Laserdiode, die mit einer für das menschliche Auge problemlosen Wellenlänge λ um 830 nm arbeitet), einen Strukturlichtempfänger in Form beispielsweise eines Infrarotlicht-Empfängers (IR-Kamera mit beispielsweise einem monochromen CMOS-Sensor), eine bevorzugt farbfähige 2D-Kamera mit einer vorbestimmten Auflösung und Bildfrequenz zur Aufnahme von 2D-Bewegt- und/oder Standbildern, und ein oder mehrere Mikrofone mit geeigneter Abtastrate und Bitbreite, beispielsweise 16 kHz und 16 Bit, zur Übertragung von Ton und Erfassung und Zuordnung von Sprachbefehlen einer Person 94 vor der Bewegungserfassungseinrichtung 100 aufweist.

Zur Entfernungsmessung kann beispielsweise von der Bewegungserfassungseinrichtung 100 bzw. dem Strukturlichtsender im Infrarotbereich strukturiertes Licht, beispielsweise in Form einer definierten Punktmatrix bzw. eines Punktgitters als bekanntes Muster, ausgestrahlt werden, welches in Erfassungsrichtung von dem Strukturlichtempfänger in der Bewegungserfassungseinrichtung 100 erfasst bzw. gemessen wird. Mit den erfassten Werten ist sodann durch Umrechnung in räumliche Koordinaten, beispielsweise mittels einer geeigneten Verarbeitungseinrichtung bzw. einem Prozessor in der Bewegungserfassungseinrichtung 100, eine Bestimmung von Entfernungen mittels Triangulation, d. h. optischer Abstandsmessung durch Winkelmessung innerhalb von Dreiecken, gegen das bekannte Muster möglich.

Bei Bewegungen einer Person 94, beispielsweise eines Anwenders wie die Dialysemaschine 60 bedienenden Personals oder eines Patienten, im Sichtbereich bzw. Erfassungsbereich der Bewegungserfassungseinrichtung 100 können durch wiederholte bzw. fortlaufende Entfernungsbestimmungsvorgänge wie vorstehend beschrieben Gesten wie beispielsweise Winken, Arme oder Beine heben, Körperdrehungen und dergleichen bis hin zur Erkennung einzelner Finger erfasst, durch entsprechende Verarbeitungsroutinen in Steuerbefehle umgesetzt und ausgegeben werden.

Derartige Steuerbefehle können an zur Interaktion mit dem Menschen vorgesehene Bedienfelder und/oder graphische Benutzeroberflächen auf Anzeigeeinrichtungen, oder Handgeräte, und/oder an Gerätekomponenten der Dialysemaschine 60 gerichtet sein.

Die Integration einer 3D-Erkennung kann somit vorteilhaft sowohl die Bedienung als auch die Bedienerunterstützung erheblich verbessern. Die 3D-Erkennung kann alternativ auch mittels einer zwei Kameras aufweisenden 3D-Kamera, einer Kombination aus Kamera und Tiefensensor oder einem dedizierten Gestensensor erfolgen, solange aus den gewonnenen Bildern, Informationen und/oder Erfassungswerten auch Tiefeninformation ableitbar ist. Ebenso ist auch eine Gestensteuerung über eine einzige 2D-Kamera oder auch über zur Erfassung von Gesten geeigneten Sensoren möglich, falls lediglich die Erkennung einfacherer Gesten erforderlich ist und Überwachungsfunktionen, die sich auf eine Auswertung der Tiefeninformation stützen, nicht benötigt werden. In anderen Worten kann die Bewegungserfassungseinrichtung 100, alternativ zur Ausführung als Kamera, auch als Sensor oder Sensoranordnung ausgebildet sein, der bzw. die eine geeignete Erkennungsfunktion ermöglicht.

Die Anordnungsposition der Bewegungserfassungseinrichtung 100 ist vorteilhaft variabel. Die Bewegungserfassungseinrichtung 100 kann an oder auf der Dialysemaschine 60 angebracht, oder in ein Gehäuse der Dialysemaschine 60 integriert sein. Alternativ ist es möglich, die Bewegungserfassungseinrichtung 100 außerhalb der und/oder entfernt von der Dialysemaschine 60 anzuordnen, beispielsweise in andere Gegenstände wie etwa das Dialysebett integriert oder an einem Infusionsständer angebracht, und dann über Kabel oder eine Vernetzung mit der Dialysemaschine 60 zu verbinden.

Zur besseren Erfassung des Patienten können eine oder mehrere zusätzliche Bewegungserfassungseinrichtungen 100 bzw. Kameras und/oder Sensoren am Fußende des Patientenbettes angeordnet sein. In diesem Fall kann es für den Erhalt eines vollständigen Bilds vorteilhaft sein, die Daten der einzelnen Erfassungseinrichtungen zu kombinieren und/oder von einer jeweils anderen Erfassungseinrichtung nicht erfassbare Bereiche für das Gesamtbild zu ergänzen.

Zur Durchführung sicherheitsrelevanter Funktionen mit der gesten- bzw. bewegungsbasierten Steuerung kann durch eine zweite Erfassungseinrichtung eine Mehrkanaligkeit mit beispielsweise einem unabhängigen Sicherheitskanal geschaffen werden.

Im Einzelnen und ohne darauf beschränkt zu sein sind durch die Bewegungserfassungseinrichtung 100 und die damit realisierbare Steuerbarkeit der Dialysemaschine 60 möglich:
a) Eine Steuerung der Dialysemaschine 60 mittels Gesten.

Während der Steuerung können auf einer Anzeigeeinrichtung bzw. einem Bildschirm der Dialysemaschine 60 unterstützende Funktionen, beispielsweise in Form von Piktogrammen, dargestellt werden. Wird sodann eine Hand einer sich im Sichtbereich der Bewegungserfassungseinrichtung 100 befindenden Person 94 erkannt, kann auf dem Bildschirm ein positionell veränderlicher ("schwebende") Zeiger dargestellt werden, etwa als Hand, die in Form und Lage der Hand der Person 94 entspricht und deren Bewegungen folgt. Mit diesem Zeiger können sodann Piktogramme nach Art von Icons am Bildschirm virtuell betätigt, oder mittels einer virtuellen Tastatur sogar Zeichen eingegeben werden.

### Beispiele für Gesten können, ohne darauf beschränkt zu sein, beinhalten:

Ein Hakenzeichen, beispielsweise gekoppelt an eine Funktion eines temporären Quittierens eines Alarmtons; eine Schiebebewegung für ein Anhalten eines Vorgangs; ein Drehen der rechten Hand mit ausgestrecktem Zeigefinger in der Luft nach rechts für beispielsweise ein Erhöhen des Blutflusses; ein Drehen der rechten Hand mit ausgestrecktem Zeigefinger in der Luft nach links für beispielsweise ein Verringern des Blutflusses; ein Drehen der linken Hand mit ausgestrecktem Zeigefinger in der Luft nach rechts für beispielsweise ein Erhöhen der Flussmenge einer Dialyseflüssigkeit; ein Drehen der linken Hand mit ausgestrecktem Zeigefinger in der Luft nach links für beispielsweise ein Verringern der Flussmenge einer Dialyseflüssigkeit; ein Winken mit allen Fingern einer Hand für beispielsweise ein Erhöhen/Senken eines Pegels; ein Wischen mit einer Hand in der Luft für beispielsweise ein Veranlassen eines Wechsels einer Bildschirmdarstellung; und/oder ein Zucken mit den Schultern für beispielsweise ein Aufrufen einer Hilfefunktion und/oder einer Bedienungsanleitung.

### b) Eine Überwachung von Bedienpersonal.

Es kann eine Identifikation der Person 94 über eine 3D-Kontur des Gesichts erfolgen, und davon abhängig verschiedene Berechtigungen zur Bedienung der Dialysemaschine 60 für die betreffende Person 94 gesetzt werden; es kann eine Überwachung von Schritten während einer Aufrüstung der Dialysemaschine 60 durchgeführt werden, einschließlich einer Einblendung spezifischer Informationen zur Unterstützung; es kann eine Identifikation von Einwegartikeln und/oder ein Vorschlagen von Standardwerten erfolgen; es kann die Punktion überwacht werden; es kann eine Überwachungsfrequenz des Personals protokolliert werden; es kann aufgezeichnet werden, ob vorbestimmte Pflegeleistungen durchgeführt werden; es kann ein Patient überwacht werden; es kann der venöse und arterielle Zugang überwacht werden; es können Krämpfe des Patienten erkannt werden; es kann die Atemfrequenz des Patienten und/oder, bei geeigneter Auflösung der Vorrichtung, der Puls überwacht werden; es können Trink- und/oder Nahrungsaufnahmevorgänge erkannt werden; es kann der Wachzustand und/oder der Schlafzustand des Patienten erkannt werden; davon abhängig können Alarmpegel gesetzt bzw. angepasst werden; es kann eine Bluterkennung durchgeführt werden; es können am Patienten, beispielsweise an dessen Zugängen, angebrachte Markierungen bzw. ein Abdecken derselben erkannt werden; und/oder es kann vom Patienten mittels Geste ein Schwesternruf ausgelöst werden.

### c) Eine Unterstützung bei Heimdialyse über Netzwerk

Es kann eine Führung des Patienten bei einem Aufrüsten der Maschine durchgeführt und eine Rückmeldung bei auftretenden Fehlern übermittelt werden; es kann eine Kommunikation mit dem Patienten durchgeführt werden; es kann eine Videokonferenz mit dem Patienten durchgeführt werden; es kann mittels Winken ein Schwesternruf ausgelöst werden, wobei ein langsames Winken beispielsweise geringe Dringlichkeit signalisiert und ein schnelles Winken hohe Dringlichkeit und/oder Komplikationen signalisiert; es können mittels pantomimischen Gesten für beispielsweise Essen oder Trinken Durst und Hunger des Patienten über Netzwerkmitteilung gemeldet werden; und/oder es können Vorort-Dienstleistungen wie beispielsweise Service, Wartung, und dergleichen unterstützt werden.

Somit wurde eine Dialysemaschine für eine Dialysebehandlung beschrieben, die zumindest eine Bewegungserfassungseinrichtung 100, die dazu angeordnet ist, in einem räumlichen Erfassungsbereich eine Bewegung einer sich in dem Erfassungsbereich befindenden Person 94 zu erfassen und Information über die erfasste Bewegung auszugeben, eine Steuereinheit 62 zur Steuerung der Dialysemaschine 60, und eine Schnittstelleneinheit 64 zur Anzeige von Betriebsinformation, zur Bedienung und/oder zur Netzwerkkommunikation der Dialysemaschine 60 beinhaltet, wobei die Steuereinheit 62 dazu angeordnet ist, auf der Grundlage der Information über die erfasste Bewegung Anweisungen zur Steuerung und/oder Regelung der Dialysemaschine 60 zu erzeugen und an steuerbare und/oder regelbare Komponenten der Dialysemaschine 60 auszugeben, und/oder Daten betreffend eine Anzeige von Betriebsinformation, eine Bedienung und/oder eine Netzwerkkommunikation mit der Schnittstelleneinheit 64 auszutauschen; und die Schnittstelleneinheit 64 dazu angeordnet ist, auf der Grundlage der ausgetauschten Daten und in Übereinstimmung mit der Information über die erfasste Bewegung die Anweisungen zur Steuerung und/oder Regelung der Dialysemaschine 60 visuell nachzuführen und/oder eine Netzwerkkommunikation durchzuführen.

Es versteht sich, dass technische Merkmale ersichtlich nicht auf hierin beschriebene, bestimmte Einrichtungen oder Vorrichtungen beschränkt sind. Insbesondere können die Bewegungserfassung und die Information über die erfasste Bewegung betreffende Verarbeitungen, Analysen, Auswertungen und dergleichen in der Bewegungserfassungseinrichtung 100, in der Steuereinheit 62 erfolgen oder auf diese Einrichtungen verteilt sein. Ebenso kann zumindest eine Speichereinrichtung zur Aufnahme und/oder Zwischenspeicherung von Daten und zumindest eine elektronische Rechen- oder Verarbeitungseinheit (Prozessor) mit Bezug zu der Bewegungserfassung in der Erfassungseinrichtung 100, der Steuereinheit 62, andernorts in der Dialysemaschine 60, und/oder in einem durch das Netzwerk 66 verbundenen Bereich vorgesehen sein.

Schließlich versteht sich, dass die Erfindung insgesamt nicht auf die konkret beschriebenen Ausführungsbeispiele beschränkt ist, sondern dass Änderungen und Modifikationen, die sich für den Fachmann anhand der Beschreibung dieser bevorzugten Ausführungsbeispiele ohne weiteres ergeben, von dem durch die nachfolgenden Patentansprüche definierten Schutzbereich umfasst sind.

### Bezugszeichenliste

- 2: Netzwerk oder Datennetzwerk
- 4: Patientenwaage
- 4a: erste Bilderfassungseinrichtung
- 6: Dialysemaschine
- 6a: zweite Bilderfassungseinrichtung
- 8: Arbeits- oder Datenstation
- 10: Server-Computer
- 60: Dialysemaschine
- 62: Steuereinheit
- 64: Schnittstelleneinheit
- 66: Netzwerk
- 70: Aufnehmer arterieller Druck
- 72: Blutpumpe
- 74: Aufnehmer venöser Druck
- 76: Bypassventil
- 78, 78a, 78b: Flusspumpe, Zulaufabschnitt, Auslaufabschnitt
- 80: Leitfähigkeitssonde
- 82: Konzentratpumpe
- 84: venöse Kammer
- 86: Dialysator
- 88: Konzentratbehälter
- 90: Aufnahme Dialysewasser
- 92: Abflussabschnitt
- 94: Person im Erfassungsbereich
- 100: Bewegungserfassungseinrichtung
- 102: Mikrofon
- 104: Lautsprecher

- S210: Start
- S211: Waage betreten
- S212: Stillstehen während Wiegen
- S213: Aufzeichnung starten
- S214: Gewicht ablesen
- S215: Gewicht dokumentieren
- S216: Protokoll
- S217: Waage verlassen
- S218: Aufzeichnung stoppen
- S219: UF Volumen errechnen
- S220: Behandlungsparameter ins Gerät übertragen

- S310: Start
- S311: Patient identifiziert sich an Waage
- S312: Waage betreten
- S313: Stillstehen während Wiegen
- S314: Aufzeichnung starten
- S315: Gewicht übertragen
- S316: Ist-Gewicht
- S317: Waage verlassen
- S318: Aufzeichnung stoppen
- S319: Kalkulation UF-Volumen (vor Dialyse)
- S320: Überprüfung ist Ist-UF (nach Dialyse)
- S321: UF Volumen als Datensatz verfügbar
- S325: Videodaten und Fotosequenz des Wiegevorgangs
- S326: Behandlungsprotokoll

- S410: Ankunft im Zentrum
- S411: Kleidung ablegen
- S412: Wiegen
- S413: Ist-Gewicht
- S414: Protokoll
- S415: Ermittlung Soll-UF (Trockengew. IST)
- S416: Therapiedaten eingeben
- S417: Dialyse mit verschriebenen Therapiedaten durchführen
- S418: Protokoll
- S419: Wiegen
- S420: Ist-Gewicht
- S421: Protokoll
- S422: Ankleiden
- S423: Übertragung in Datenbank
- S424: Zentrum verlassen

## Patentansprüche

1. Dialysemaschine für eine Dialysebehandlung, mit
zumindest einer Bewegungserfassungseinrichtung (100), die dazu angeordnet ist, in einem räumlichen Erfassungsbereich eine Bewegung einer sich in dem Erfassungsbereich befindenden Person (94) zu erfassen und Information über die erfasste Bewegung auszugeben;
einer Steuereinheit (62) zur Steuerung der Dialysemaschine (60); und
einer Schnittstelleneinheit (64) zur Anzeige von Betriebsinformation, zur Bedienung und/oder zur Netzwerkkommunikation der Dialysemaschine (60), wobei
die Steuereinheit (62) dazu angeordnet ist, auf der Grundlage der Information über die erfasste Bewegung Anweisungen zur Steuerung der Dialysemaschine (60) zu erzeugen und an steuerbare Komponenten der Dialysemaschine (60) auszugeben und Daten betreffend eine Anzeige von Betriebsinformation, eine Bedienung und/oder eine Netzwerkkommunikation mit der Schnittstelleneinheit (64) auszutauschen; und
die Schnittstelleneinheit (64) dazu angeordnet ist, auf der Grundlage der ausgetauschten Daten und in Übereinstimmung mit der Information über die erfasste Bewegung die Anweisungen zur Steuerung der Dialysemaschine (60) visuell nachzuführen und/oder eine Netzwerkkommunikation durchzuführen,
**gekennzeichnet durch**
eine Gesichtserkennungsfunktion und/oder Markierungserkennungsfunktion als eine Funktion zur Unterscheidung mehrerer sich in dem Erfassungsbereich befindender Personen, wobei die Dialysemaschine dazu angeordnet ist, mittels der Gesichtserkennungsfunktion und/oder der Markierungserkennungsfunktion eine sich in dem Erfassungsbereich befindende Person zu klassifizieren und der Person entsprechend ihrer Klassifikation zulässige Bewegungen und/oder zulässige Bedienvorgänge zuzuordnen.

2. Dialysemaschine nach Anspruch 1, bei der die Bewegungserfassungseinrichtung (100) eine 3D-Kameraanordnung zur Erfassung von Bildinformation in drei Dimensionen ist, die Strukturlicht mit einem vorbestimmten Lichtmuster in den räumlichen Erfassungsbereich ausstrahlt und wieder empfängt, auf der Grundlage eines Vergleichs und/oder einer Auswertung des ausgestrahlten Lichtmusters und des empfangenen Lichtmusters Tiefeninformation enthaltende Bildinformation ermittelt, und daraus die Information über die erfasste Bewegung erzeugt.

3. Dialysemaschine nach Anspruch 1, bei der die Bewegungserfassungseinrichtung (100) eine 2D-Kamera aufweist, die dazu angeordnet ist, zweidimensionale 2D-Bildinformation zu erfassen, die als Bewegt- und/oder Standbild mittels Netzwerkkommunikation über ein Netzwerk (66) an eine entfernte Stelle übertragbar ist.

4. Dialysemaschine nach Anspruch 1, bei der die Bewegungserfassungseinrichtung (100) eine 2D-Kamera zur Erfassung von Bildinformation in zwei Dimensionen aufweist, die dazu ausgelegt ist, die Information über die erfasste Bewegung zu erzeugen.

5. Dialysemaschine nach Anspruch 1, bei der die Bewegungserfassungseinrichtung (100) eine Sensoreinrichtung ist, die dazu ausgelegt ist, in einem Erfassungsbereich der Sensoreinrichtung eine Bewegung zu erkennen.

6. Dialysemaschine nach einem der vorangehenden Ansprüche, bei der die erfasste Bewegung eine Geste einer sich in dem Erfassungsbereich befindenden Person ist.

7. Dialysemaschine nach einem der vorangehenden Ansprüche, bei der die Bewegungserfassungseinrichtung (100) in ein die Dialysemaschine (60) umschließendes Gehäuse integriert ist.

8. Dialysemaschine nach einem der vorangehenden Ansprüche, bei der zumindest eine Bewegungserfassungseinrichtung (100) außerhalb eines die Dialysemaschine (60) umschließenden Gehäuses angeordnet ist.

9. Dialysemaschine nach Anspruch 1, die dazu angeordnet ist, mittels der Funktion zur Unterscheidung mehrerer sich in dem Erfassungsbereich befindender Personen der Person entsprechend ihrer Klassifikation eine in Bezug auf eine aus einer Bewegung ableitbare Dringlichkeit abgestufte Bedeutung einer Bewegung zuzuordnen.

10. Dialysemaschine nach einem der vorangehenden Ansprüche, bei der eine von der Bewegungserfassungseinrichtung (100) in dem Erfassungsbereich erkannte Bewegung derart auf eine berührungsempfindliche Anzeigeeinrichtung der Dialysemaschine gespiegelt wird, dass auf der Anzeigeeinrichtung eine mittels der Bewegung veranlasste und/oder ausgeführte Funktion der Dialysemaschine (60) analog zu einer auf Berühren der Anzeigeeinrichtung erfolgenden Veranlassung und/oder Ausführung visuell nachgeführt, nachgebildet und/oder bestätigt wird.

## Claims

1. Dialysis machine for a dialysis treatment, with
at least one movement detection device (100), adapted to detect in a spatial detection region a movement of a person (94) present in the detection region and to output information or the detected movement;
a control unit (62) for controlling the dialysis machine (60); and
an interface unit (64) for displaying operating information, for operating and/or for network communication of the dialysis machine (60), wherein
the control unit (62) is adapted to generate instructions for controlling the dialysis machine (60) based on the information on the detected movement and to output them to controllable components of the dialysis machine (60) and to exchange data concerning displaying operating information, an operation and/or a network communication with the interface unit (64); and
the interface unit (64) is adapted to visually track the instructions for controlling the dialysis machine (60) based on the exchanged data and in accordance with the information on the detected movement and/or to perform a network communication,
**characterized by**
a face recognition function and/or mark recognition function as a function for distinguishing several persons present in the detection region, wherein the dialysis machine is adapted to classify a person present in the detection region via the face recognition function and/or mark recognition function and to assign admissible movements and/or admissible operation processes in correspondence with the person's classification.

2. Dialysis machine according to claim 1, in which the movement detection device (100) is a 3D camera assembly for detecting image information in three dimensions, which emits and receives structured light with a predetermined light pattern in the spatial detection region, determining image information containing depth information based on a comparison and/or an evaluation of the emitted light pattern and the received light pattern and generating therefrom the information on the detected movement.

3. Dialysis machine according to claim 1, in which the movement detection device (100) comprises a 2D camera which is adapted to detect two-dimensional 2D image information which can be transferred as moving image and/or still image from a network (66) to a remote place via network communication.

4. Dialysis machine according to claim 1, in which the movement detection device (100) comprises a 2D camera for detecting image information in two-dimensions which is configured to generate the information on the detected movement.

5. Dialysis machine according to claim 1, in which the movement detection device (100) is a sensor device which is configured to detect a movement in a detection region of the sensor device.

6. Dialysis machine according to one of the preceding claims, in which the detected movement is a gesture of a person present in the detection region.

7. Dialysis machine according to one of the preceding claims, in which the movement detection device (100) is integrated into a housing surrounding the dialysis machine (60).

8. Dialysis machine according to one of the preceding claims, in which at least one movement detection device (100) is arranged outside of the housing surrounding the dialysis machine (60).

9. Dialysis machine according to claim 1, which is adapted to assign a graded importance of a movement with regard to an urgency derivable from a movement to a person corresponding to their classification via the function for distinguishing several persons present in the detection region.

10. Dialysis machine according to one of the preceding claims, in which a movement detected by the movement detection device (100) in the detection region is mirrored to a touch-sensitive display device of the dialysis machine in such a way that a function of the dialysis machine (60) induced and/or executed on the display device via the movement is visually tracked, copied and/or confirmed analogously to an inducement and/or execution as response to touching of the display device.

## Revendications

1. Machine de dialyse pour un traitement par dialyse, avec
au moins un dispositif de détection de mouvement (100), qui est agencé pour détecter dans une zone de détection spatiale un mouvement d'une personne (94) se trouvant dans la zone de détection et émettre une information sur le mouvement détecté ;
une unité de commande (62) pour la commande de la machine de dialyse (60) ; et une unité d'interface (64) pour l'affichage d'une information de service, pour la commande et/ou pour la communication de réseau de la machine de dialyse (60), dans laquelle
l'unité de commande (62) est agencée pour générer, sur la base de l'information sur le mouvement détecté, des instructions pour la commande de la machine de dialyse (60) et pour les émettre à des composants pouvant être commandés de la machine de dialyse (60) et pour échanger des données concernant un affichage d'informations de service, une commande et/ou une communication de réseau avec l'unité d'interface (64) ; et
l'unité d'interface (64) est agencée pour suivre visuellement, sur la base des données échangées et en correspondance avec l'information sur le mouvement détecté, les instructions pour la commande de la machine de dialyse (60) et/ou pour effectuer une communication de réseau,
**caractérisée par**
une fonction de reconnaissance faciale et/ou fonction de détection de marquage en tant que fonction pour la distinction de plusieurs personnes se trouvant dans la zone de détection, dans laquelle la machine de dialyse est agencée pour classifier, au moyen de la fonction de reconnaissance faciale et/ou de la fonction de détection de marquage, une personne se trouvant dans la zone de détection et pour affecter des mouvements autorisés et/ou des opérations de commande autorisés à la personne en fonction de sa classification.

2. Machine de dialyse selon la revendication 1, dans laquelle le dispositif de détection de mouvement (100) est un ensemble de caméras 3D pour la détection d'information d'image en trois dimensions, qui émet et reçoit à nouveau une lumière structurée avec un motif de lumière prédéterminé dans la zone de détection spatiale, sur la base d'une comparaison et/ou d'une évaluation du motif de lumière émis et du motif de lumière reçu, détermine une information d'image contenant une information de profondeur, et génère à partir de là l'information sur le mouvement détecté.

3. Machine de dialyse selon la revendication 1, dans laquelle le dispositif de détection de mouvement (100) présente une caméra 2D, qui est agencée pour détecter une information d'image 2D en deux dimensions, qui peut être transférée en tant qu'image animée et/ou image fixe à un poste éloigné au moyen de la communication de réseau par le biais d'un réseau (66).

4. Machine de dialyse selon la revendication 1, dans laquelle le dispositif de détection de mouvement (100) présente une caméra 2D pour la détection d'informations image en deux dimensions, qui est conçue pour générer l'information sur le mouvement détecté.

5. Machine de dialyse selon la revendication 1, dans laquelle le dispositif de détection de mouvement (100) est un dispositif de capteur, qui est conçu pour détecter un mouvement dans une zone de détection du dispositif de capteur.

6. Machine de dialyse selon l'une quelconque des revendications précédentes, dans laquelle le mouvement détecté est un geste d'une personne se trouvant dans la zone de détection.

7. Machine de dialyse selon l'une quelconque des revendications précédentes, dans laquelle le dispositif de détection de mouvement (100) est intégré à un boîtier entourant la machine de dialyse (60).

8. Machine de dialyse selon l'une quelconque des revendications précédentes, dans laquelle au moins un dispositif de détection de mouvement (100) est agencé à l'extérieur d'un boîtier entourant la machine de dialyse (60).

9. Machine de dialyse selon la revendication 1, qui est agencée pour affecter, au moyen de la fonction de distinction de plusieurs personnes se trouvant dans la zone de détection, une importance échelonnée d'un mouvement par rapport à une urgence pouvant être déduite d'un mouvement à la personne en fonction de sa classification.

10. Machine de dialyse selon l'une quelconque des revendications précédentes, dans laquelle un mouvement détecté par le dispositif de détection de mouvement (100) dans la zone de détection est dupliqué sur un dispositif d'affichage tactile de la machine de dialyse de telle sorte que sur le dispositif d'affichage une fonction de la machine de dialyse (60) ordonnée et/ou réalisée au moyen du mouvement est suivie visuellement, reproduite et/ou confirmée de façon analogue à un ordre et/ou une réalisation se faisant par toucher du dispositif d'affichage.
